# EUROPEAN PATENT SPECIFICATION

(11) **EP 1 023 081 B1**
(45) Date of publication and mention of the grant of the patent: **03.10.2007**
(21) Application number: 98925036.0
(22) Date of filing: 28.05.1998
(51) Int. Cl.: A61K 38/17, A61P 11/00, G01N 33/50

(54) **USE OF RECOMBINANT HUMAN UTEROGLOBIN IN TREATMENT OF FIBROTIC CONDITIONS**
VERWENDUNG VON REKOMBINANTEN MENSCHLICHEN UTEROGLOBIN ZUR BEHANDLUNG VON FIBROTISCHEN KRANKHEITSZUSTÄNDEN
UTILISATION DE L'UTEROGLOBINE DE RECOMBINAISON HUMAINE DANS LE TRAITEMENT DES FIBROSES

(30) Priority: 28.05.1997 US 864357
(43) Date of publication of application: 02.08.2000
(73) Proprietor: Claragen, Inc., College Park, MD 20742 (US); NATIONAL INSTITUTE OF HEALTH, Rockville, MD 20852 (US)
(72) Inventor: PILON, Aprile, L., Gaithersburg, MD 20879 (US); MUKHERJEE, Anil, B., Brookeville, MD 20833-0136 (US); ZHANG, Zhongjian, Rockville, MD 20850 (US)
(74) Representative: Naylor, Kathryn May
(86) International application number: PCT/US1998/011026
(87) International publication number: WO 1998/053846

(56) References cited:
- WO-A-88/08983
- WO-A-96/40657
- US-A- 5 266 562
- GUY J ET AL: "Surfactant-producing rabbit pulmonary alveolar type II cells synthesize and secrete an antiinflammatory protein, uteroglobin." BIOCHEMICAL AND BIOPHYSICAL RESEARCH COMMUNICATIONS, vol. 189, no. 2, 15 December 1992 (1992-12-15), pages 662-669, XP002153573
- PERI A ET AL: "Uteroglobin gene expression in the rabbit uterus throughout gestation and in fetal lung: Relationship between uteroglobin and eicosanoid levels in the developing fetal lung." JOURNAL OF CLINICAL INVESTIGATION, vol. 96, no. 1, July 1995 (1995-07), pages 343-353, XP000960725
- CHAN C-C ET AL: "Effects of antiflammins on endotoxin-induced uveitis in rats" ARCHIVES OF OPHTHALMOLOGY, vol. 109, no. 2, February 1991 (1991-02), pages 278-281, XP000960732
- MUKHERJEE A B ET AL: "Regulation of extracellular phospholipase A2 activity: Implication for inflammatory diseases" DNA AND CELL BIOLOGY, vol. 11, no. 3, April 1992 (1992-04), pages 233-243, XP000960988
- DIERYNCK I ET AL: "The human Clara cell protein: biochemical and biological characterization of a natural immunosuppressor" MULTIPLE SCLEROSIS, vol. 1, no. 6, 1996, pages 385-387, XP000990474
- ZHANG ZH ET AL: "Human uteroglobin gene: Structure, subchromosomal localization, and polymorphism." DNA AND CELL BIOLOGY, vol. 16, no. 1, January 1997 (1997-01), pages 73-83, XP000960734
- VAN VYVE T ET AL: "Protein content in bronchoalveolar lavage fluid of patients with asthma and control subjects." JOURNAL OF ALLERGY AND CLINICAL IMMUNOLOGY, vol. 95, no. 1 Part 1, January 1995 (1995-01), pages 60-68, XP000990403
- MUKHERJEE A B ET AL: "Could the gene coding for human uteroglobin (Clara Cell 10kDa protein) be a candidate gene for atopy?" AMERICAN JOURNAL OF HUMAN GENETICS, vol. 55, no. 3, Supplement, September 1994 (1994-09), page A197 XP000960727
- ZHANG ZH ET AL: "Severe fibronectin-deposit renal glomerular disease in mice lacking uteroglobin." SCIENCE, vol. 276, no. 5317, 30 May 1997 (1997-05-30), pages 1408-1412, XP002153574
- CHIESA M ET AL: "Significant increase in immunoregulatory protein blastokinin/uteroglobin in IgA/fibronectin complexes in sera of patients with IgA nephropathy" NEPHROLOGY DIALYSIS TRANSPLANTATION, vol. 15, no. 9, 2000, page A39 XP000971167
- LEYTON et al., "Recombinant Human Uteroglobin Inhibits the In Vitro Invasiveness of Human Metastatic Prostate Tumor Cells and the Release of Arachidonic Acid Stimulated by Fibroblast-Conditioned Medium1", CANCER RES., 15 July 1994, Vol. 54, pages 3696-3699, XP002910194
- EDELSON et al., "Acute Lung Injury Induced by Phospholipase A2: Structural and Functional Changes1-3", AM. REV. RESPIR. DIS., May 1991, Vol. 143, pages 1102-1109, XP002910195

## Description

### FIELD OF THE INVENTION

The invention relates generally to the treatment of inflammatory and fibrotic conditions using native human uteroglobin (hUG) or recombinant human uteroglobin (rhUG). Novel physiological roles and therapies for UG (hUG or rhUG) have been identified. Specifically, the invention relates to the treatment of inflammatory and fibrotic conditions by administering hUG or rhUG to inhibit PLA₂s and/or to prevent fibronectin deposition. The invention further provides for the treatment of neonatal respiratory distress syndrome (RDS) and bronchopulmonary dysplasia (BPD), a critical clinical condition of the lung, and glomerular nephropathy, a disease of the kidney, both characterized by the inflammatory and fibrotic conditions.

Documents cited in this application relate to the state-of-the-art to which this invention pertains.

### BACKGROUND OF THE INVENTION

### Inflammatory and Fibrotic Conditions

The search for improved therapeutic agents for the treatment of inflammatory and fibrotic diseases has received much attention in recent years. Neonatal RDS, a lung surfactant deficiency disease, is a condition of particular interest in that it is one of the major causes of mortality in premature neonates. While introduction of surfactant therapy dramatically improves survival of RDS patients, the development of chronic inflammatory and fibrotic disease in a significant percentage of this patient population is a major problem. Likewise, hereditary fibronectin-deposit glomerular nephropathy leads to end stage renal failure when patients' kidneys become blocked and no longer filter the blood. Nephropathy is characterized by fibronectin deposits and fibrosis of the kidneys which render the organ non-functional, and eventually, unable to support life.

PLA₂ (phospholipase A₂), a class of endogenous enzymes that hydrolyze the Sn2 position ester bond of glycerophospholipids, is one of many proteins implicated in inflammatory and fibrotic conditions. It is also responsible for hydrolysis of surfactant phospholipids in the lungs. Uteroglobin (also known as CC10, CC16, CC 17, urine protein-1, P-1, progesterone binding protein, PCB-binding protein, Clara cell secretory protein (CCSP), blastokinin, retinol-binding protein, phospholipid-binding protein, and alpha2-microglobulin) inhibits the activity of PLA₂ *in vitro*.

Uteroglobin is a small globular homodimeric protein. It has a molecular weight of 15.8 kDa, but it migrates in electrophoretic gels at a size corresponding to 10 kDa. Human uteroglobin is abundant in the adult human lung, and comprises up to about 7% of the total soluble protein. However, its expression is not fully activated in the developing human fetus until late in gestation. Consequently, the extracellular lung fluids of pre-term infants contain far less UG than those of adults. UG is also expressed by the pancreas.

PLA₂s play critical roles in the inflammatory response because they release arachidonic acid (AA) from cellular phospholipid reservoirs. AA is metabolized to a number of potent inflammatory mediators in a process referred to as the arachidonic acid cascade.

Several acute and chronic clinical conditions have been characterized by elevated serum or local PLA₂ activity (see Table 1, below).

**Table 1. Clinical Conditions Associated with PLA₂ Activity**

| Diseases | Sites |
|---|---|
| Rheumatoid arthritis | Serum, synovial fluid, WBC |
| Collagen vascular diseases | Serum |
| Pancreatitis | Serum |
| Peritonitis | Peritoneal fluid and cells |
| Septic shock | Serum |
| ARDS^{a} | Serum and alveolar fluid |
| Acute renal failure | Serum |
| Autoimmune uveitis | Serum, aqueous humor |
| Bronchial asthma | Bronchial fluid |

| | |
|---|---|
| ^{a} Adult respiratory distress syndrome | |

There are no effective PLA₂ inhibitors presently available for clinical use. To date, only a few PLA₂ inhibitors have progressed into clinical trials, but none have qualified for commercial marketing.

Fibronectin (Fn) is a 200 kDa glycoprotein which exists in several different forms and is secreted by different tissues. Fn is an essential protein and targeted disruption of the Fn gene in mice showed that it has a central role in embryogenesis. Fn also plays a key role in inflammation, cell adhesion, tissue repair and fibrosis, and is deposited at the site of injury. Plasma fibronectin (pFn) is secreted by the liver and circulates in the plasma. In the lung, cellular Fn (cFn) is secreted upon inflammation and injury. Both types of Fn are chemotactic factors for inflammatory cells and fibroblasts. Large numbers of inflammatory cells and fibroblasts infiltrate the lung during inflammatory episodes, which can lead to pulmonary fibrosis and ultimately death. Elevated levels of Fn have been detected in human clinical conditions such as neonatal RDS and BPD of the lung, and glomerular nephropathy of the kidney.

### The Role of UG

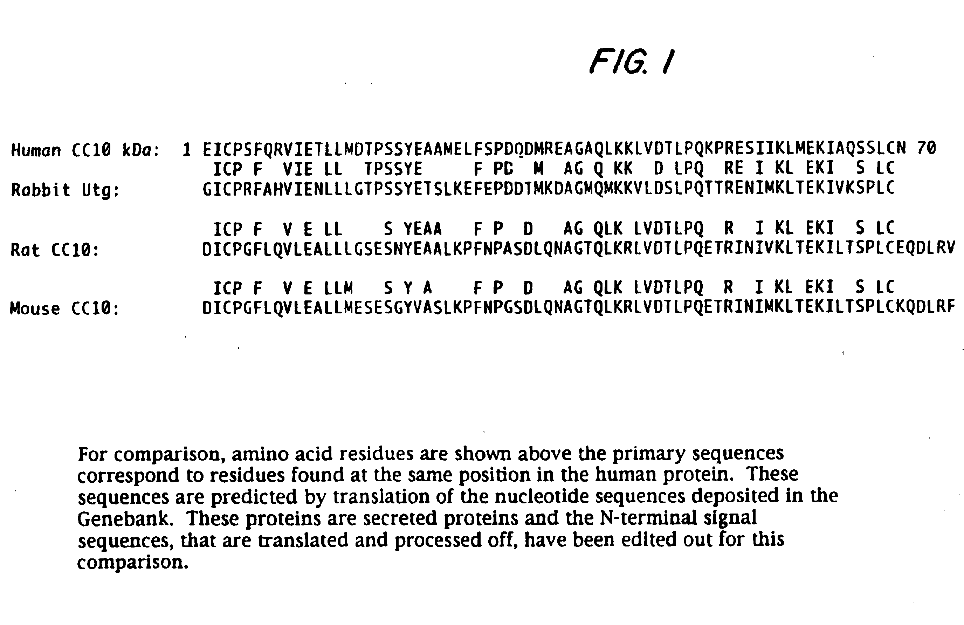

Amino acid analysis of purified human UG reveals that it is structurally similar but not identical to other "UG-like" proteins, e.g. rabbit UG. 39 of 70 amino acids are identical between human and rabbit UG (see Figure 1). The "UG-like" proteins, including human UG/CC10, rat CC10, mouse CC10, and rabbit UG, exhibit species-specific and tissue-specific antigenic differences, as well as differences in their tissue distribution and biochemical activities *in vitro.* UG-like proteins have been described in many different contexts with regard to tissue and species of origin, including rat lung, human urine, sputum, blood components, rabbit uterus, rat and human prostate, and human lung. At present there are no known physiological roles for these proteins.

Despite years of study, the biological roles of these proteins *in vivo* remain unclear. The absence of structural identity among UG-like proteins makes it impossible to predict whether a protein will possess *in vivo* therapeutic function in humans based on *in vitro* or other activity exhibited by a structurally related protein. For example, human uteroglobin binds less than 5% of the amount of progesterone as rabbit UG binds in the same assay. Human UG has a lower isoelectric point (4.6) than rabbit UG (5.4).

Stripp *et al*. (1996) have reported studies on a uteroglobin knockout mouse generated to eliminate expression of uteroglobin. The mouse has Clara cells which exhibit odd intracellular structures in place of uteroglobin secretion granules, but there is no other phenotype. This observation is highly significant because pulmonary function accompanied by pulmonary inflammation and fibrosis was expected. Moreover, this knockout mouse showed no evidence of renal, pancreatic, or reproductive abnormality, indicating that the uteroglobin protein had no significant role in controlling inflammation or fibrosis *in vivo*.

### OBJECTS OF THE INVENTION

Therefore, it is an object of the invention to provide a pharmaceutical composition including a fibronectin binding effective amount of recombinant human uteroglobin or a fragment or derivative thereof, a lung surfactant and a pharmaceutically acceptable carrier or diluent.

Further, the invention discloses a method for inhibiting PLA₂ enzymes *in vivo* in a mammal in need of such treatment, wherein the method includes administering to a mammal a PLA₂ inhibiting effective amount of native or recombinant human uteroglobin or a fragment or derivative thereof.

Still further, the present invention discloses a method for treating or preventing an inflammatory condition in a patient in need of such treatment, wherein the method includes administering an anti-inflammatory effective amount of native or recombinant UG or a fragment or derivative thereof.

It is an object of the present invention to provide a use, for the manufacture of a pharmaceutical composition for treating or preventing a fibrotic condition in a patient in need of such treatment, of a fibronectin binding effective amount of native or recombinant UG or a fragment or derivative thereof.

It is an additional object of the present invention to provide a use, for the manufacture of a pharmaceutical composition for treating or preventing an inflammatory or fibrotic condition characterized by a deficiency of endogenous UG, of a compensating amount of native or recombinant UG or a fragment or derivative thereof.

Finally, it is an object of the present invention to provide an assay quantitating uteroglobin-fibronectin complexes in a clinical sample.

### SUMMARY OF THE INVENTION

It has now been found that uteroglobin plays a central physiological role in inhibition of PLA₂s and in prevention of fibronectin deposition and fibrosis *in vivo.* A combination of experiments performed in a new strain of transgenic uteroglobin "knockout" mice, and in a monkey model of neonatal respiratory distress syndrome (RDS) which involves pulmonary inflammation and fibrosis demonstrate these effects. The uteroglobin knockout mice of the present invention (hereinafter the "UG KO mice/mouse") exhibit lethal glomerular nephropathy and renal parenchymal fibrosis, as early and late onset diseases, respectively. Administration of exogenous Fn to normal mice causes Fn deposition in the kidneys, but administration of equimolar amounts of Fn and rhUG does not.

Reduction of PLA₂ activity *in vivo* has been demonstrated in the presence of rhUG. In a first experiment, the phenotype of the UG KO mice revealed that serum PLA₂ activity is significantly elevated in the absence of UG, compared to PLA₂ activity in littermates possessing a functional UG gene. In a second experiment, administration of rhUG to pre-term monkeys suffering from RDS was shown to inhibit PLA₂ activity in the extracellular fluids of the lungs.

Other experiments demonstrate that *in vitro* PLA₂ can degrade the artificial surfactant (typically Survanta) used in treatment of RDS and that UG can inhibit this degradation. These experiments demonstrate that UG mediates PLA₂ inhibition and Fn deposition *in vivo* following intratracheal or intravenous administration.

Experiments with the uteroglobin knockout mouse demonstrate that rhUG may be used to treat conditions in which uteroglobin is found to be deficient or the protein itself bears a loss-of-function mutation. It has now been discovered that rhUG may be used to treat or prevent inflammatory or fibrotic conditions in which functional endogenous uteroglobin is deficient in the circulation or at the site of inflammation or fibrosis. Reductions in the levels of hUG in serum and/or broncho-alveolar lavage fluids have been found in certain pulmonary inflammatory or fibrotic conditions, including pre-term infants at risk for developing neonatal BPD. It has been found that UG may be used to supplement deficient or defective endogenous uteroglobin to prevent or treat such inflammatory and fibrotic conditions.

According to one aspect, the invention discloses a method of treating an inflammatory condition *in vivo* comprising administering to a patient in need of such treatment an anti-inflammatory effective amount of UG.

According to a further aspect, the invention discloses a method of inhibiting soluble PLA₂ enzymes *in vivo*, which comprises administering to a patient in need of such treatment a PLA₂ inhibiting effective amount of UG.

The invention provides a use, for the manufacture of a pharmaceutical composition for treating or preventing a fibrotic condition, of a fibronectin binding effective amount of UG.

A further aspect of the invention provides a use, for the manufacture of a pharmaceutical composition for treating or preventing fibrillogenesis of a fibronectin binding amount of UG.

In accordance with a further aspect, the invention provides a use, for the manufacture of a pharmaceutical composition for treating or preventing a fibrotic condition characterized by a deficiency of endogenous functional UG, of a compensating amount of UG.

The invention also provides pharmaceutical compositions comprising an effective amount of rhUG in association with a lung surfactant and a pharmaceutically acceptable carrier or diluent. The compositions may take the form of injectable solutions, and liquids or semi-aerosols for intratracheal administration.

According to a further aspect, the invention provides pharmaceutical compositions comprising rhUG and a lung surfactant (e.g. Survanta (a bovine lung extract from Abbott Labs) and Exosurf (a chemically synthetic lung surfactant from Glaxo-Wellcome)) in association with a pharmaceutically acceptable carrier or diluent.

In another aspect, the invention provides an assay for quantitating uteroglobin-fibronectin complexes in a clinical sample, wherein a clinical sample suspected of containing a uteroglobin-fibronectin complex is contacted with an antigen capture agent, for example a monospecific rabbit polyclonal antibody, immobilized on an insoluble support; an antigen detection agent, for example an antibody specific for fibronectin, is added to the sample; and the presence of any complex bound to the support is detected using, for example, a secondary antibody, e.g., anti-IgG antibody conjugated to an enzyme such as horse radish peroxidase using a standard enzymatic reaction wherein the enzyme substrate is converted to a chromogenic or fluorogenic compound which is quantitated using standard spectrophotometric or fluorometric apparatus.

### BRIEF DESCRIPTION OF THE DRAWINGS

The invention will now be described in more detail, with reference to the accompanying drawings, in which:
FIGURE 1 shows an alignment of UG-like proteins;

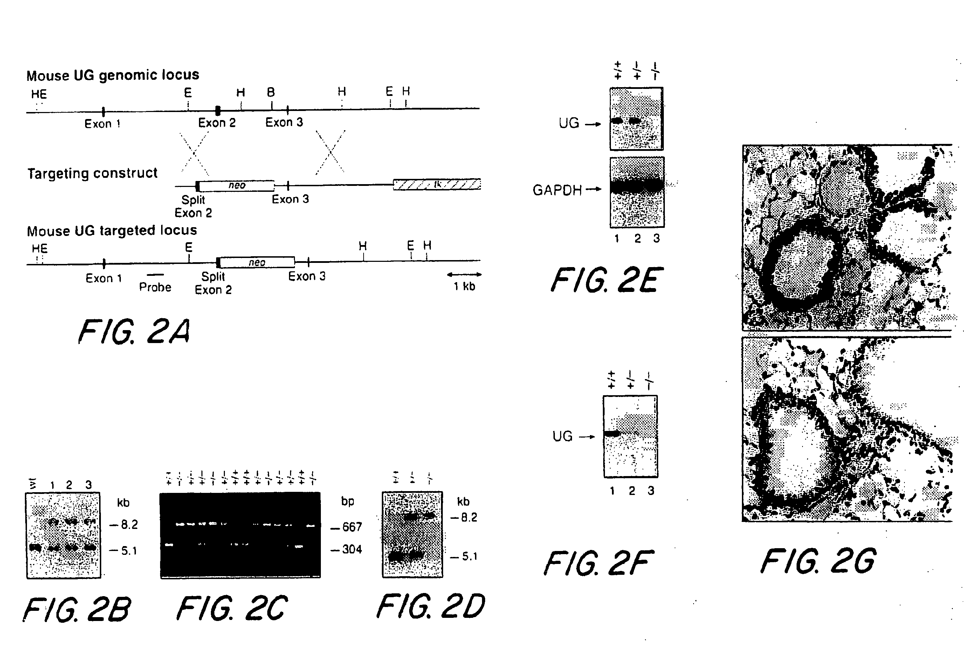

FIGURE 2A shows the intended targeting construct of the transgenic UG knockout mouse; the restriction sites are B - BamIII, E = EcoRI, H = HindIII; FIGURES 2B-2D show verification of the genetic construct in progeny of transgenic embryos by PCR and Southern blot analyses; (B) southern blot analyses of the targeted ES R1 cell clones, wherein Wt = wild type; (C) representative PCR analyses of genomic DNA from tail biopsies of offspring; the genotypes and their corresponding PCR products are as follows: UG^{+/+}, 304 bp; UG^{+/-}, 304 and 667 bp; UG^{-/-}, 667 bp; (D) southern blot of mouse tail genomic DNA; FIGURE 2E shows confirmation of the absence of UG-mRNA in the lung tissues of UG^{-/-} mice by RT-PCR analysis; RT-PCR analyses of total RNA extracted from the lung tissues of littermates with UG^{+/+}, UG^{+/-}, and UG^{-/-} genotypes; a 273 bp RT-PCR product was detectable in the lungs of UG^{+/+} and UG^{+/-} but lacking from those of UG^{-/-} mice; FIGURE 2F shows confirmation of the absence of UG protein in the lungs of UG^{-/-} mice by Western analysis; proteins (30 micrograms each) from lung lysate were resolved by electrophoresis using 4-20% gradient SDS-Page under non-reducing conditions and immunoblotted using rabbit anti-mouse UG; FIGURE 2G shows confirmation of the absence of UG in lung tissue sections of the UG^{-/-} mice using immunohistochemical methods in bronchiolar epithelial cells; the dark staining over the bronchiolar epithelial cells of UG^{+/+} mouse (upper panel) indicates UG immunoreactivity; note the absence of immunoreactivity in UG^{-/-} mouse lungs (lower panel).

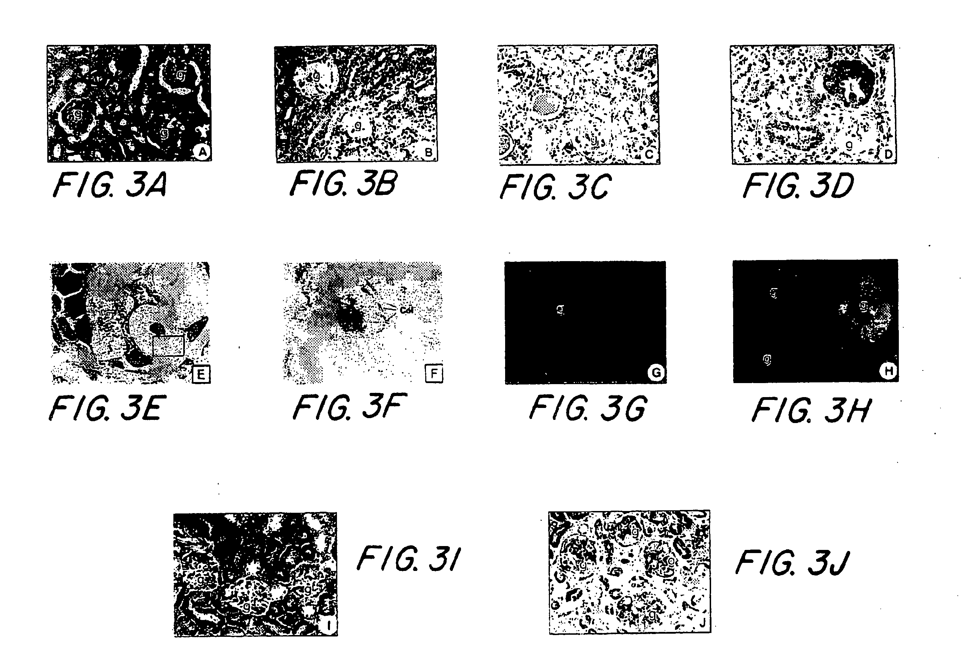

FIGURES 3A-3J compare histopathological analyses of kidney sections from normal versus UG^{-/-} mice, showing abnormal parenchymal fibrosis and glomerular Fn deposition in the knockout mice only; H & E staining of kidney sections from a UG^{+/+} (A) and its UG^{-/-} (B) littermate; (C) kidney section of a 10 month old mouse with severe parenchymal fibrosis; (D) a region of the same mouse kidney in (C) showing renal tubular hyperplasia (magnification 40X, g = glomerulus; f = fibrosis; t = tubule); (E) transmission electron microscopy of the glomerular deposit of a UG^{-/-} mouse with severe renal disease (magnification 6000X); (F) the inset in (E) is magnified 60.000X, which shows the long striated fibrillar structures indicative of collagen (col) and short diffuse ones consistent with Fn fibrils; (G) Fn immunofluorescence of a kidney section from a UG^{+/+} mouse using murine Fn-antibody; (H) Fn-immunofluorescence of a kidney section from a UG^{-/-} mouse with severe renal disease; Mason's trichrome staining of the kidney sections with UG^{+/+} (I) and UG^{-/-} (J) mice; the bluish staining over the glomeruli of UG^{-/-} mouse kidney section is collagen (magnification approximately 40X).

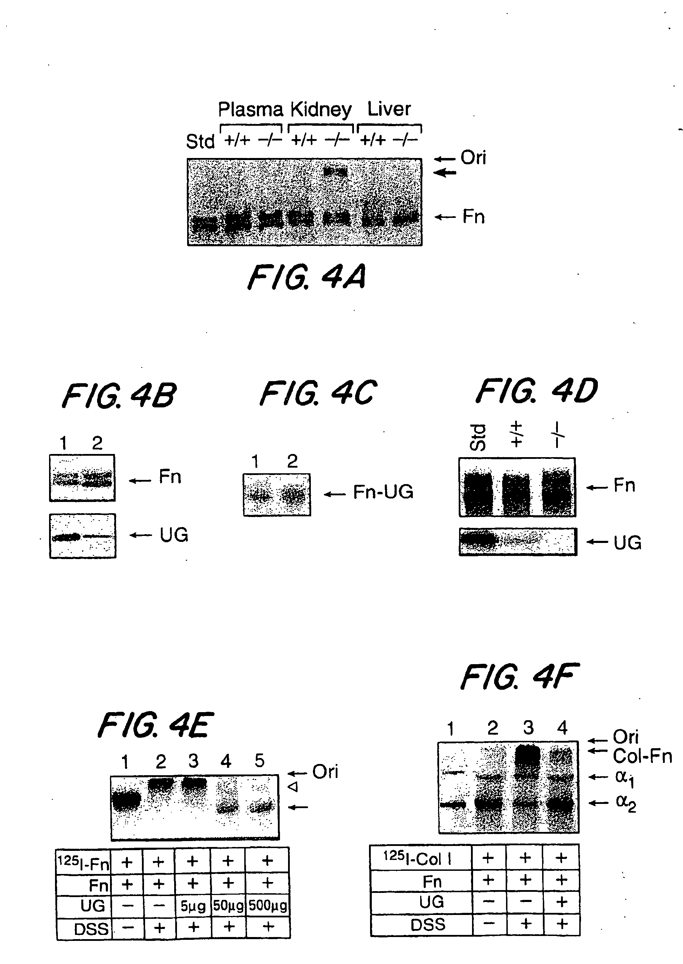

FIGURE 4A shows the presence of Fn aggregates only in the kidneys of the UG^{-/-} mice; immunoprecipitation and western blotting of Fn from plasma, kidney, and liver of UG^{+/+} and UG^{-/-} mice; a multimeric Fn band (bold arrow) was detected only in the kidney lysates of UG^{-/-} mice.
FIGURES 4B and 4C show the formation of UG-Fn complexes *in vitro*; (B) equimolar concentrations of UG and Fn were inclubated, immunoprecipitated with and detected by Western blotting with either Fn or UG antibody; the immunoprecipitates contain both Fn (lane 2, upper panel) and UG (lane 2, lower panel); lanes 1 of both panels represent Fn and UG standards; (C) equimolar concentrations of ¹²⁵I-UG and Fn were incubated at 4C for 1 hour and the resulting complex was resolved by electrophoresis on 6% non-reducing, non-denaturing polyacrylamide gels; lane 1, coomassie blue stained Fn-UG heteromer; lane 2, its autoradiogram.
FIGURE 4D shows the presence of UG-Fn complexes in the plasma of normal but not UG^{-/-} mice; immunoprecipitation of plasma from UG^{+/+} and UG^{-/-} mice with Fn-antibody and western blotting with Fn and UG antibodies; Fn (upper panel); UG (lower panel); std = standards for UG and Fn.
FIGURE 4E shows the dose-dependent inhibition of Fn self-aggregation by UG in vitro; affinity-crosslinking of ¹²⁵I-Fn with unlabeled Fn in the absence (lane 2) and presence of varying amounts of UG (lanes 3-5); the intensity of the very high molecular weight, radioactive Fn band (land 2) formed in the absence of UG is reduced in a dose-dependent manner; lane 1, ¹²⁵I-Fn with unlabeled Fn in the absence of UG and DSS; open arrowhead - multimeric Fn; lower thin arrow = 220 kDa Fn.
FIGURE 4F shows the inhibition of Fn-collagen complex formation by UG; affinity crosslinking of ¹²⁵I-collagen I with unlabeled Fn in the absence of (lane 3) and presence (lane 4) of UG; lane 1, coomassie blue-stained collagen I; alpha₁-alpha₁ chain of collagen I and alpha₂-alpha₂ chain of collagen I; lane 2, ¹²⁵I-collagen I and unlabeled Fn in the absence of UG and DSS.

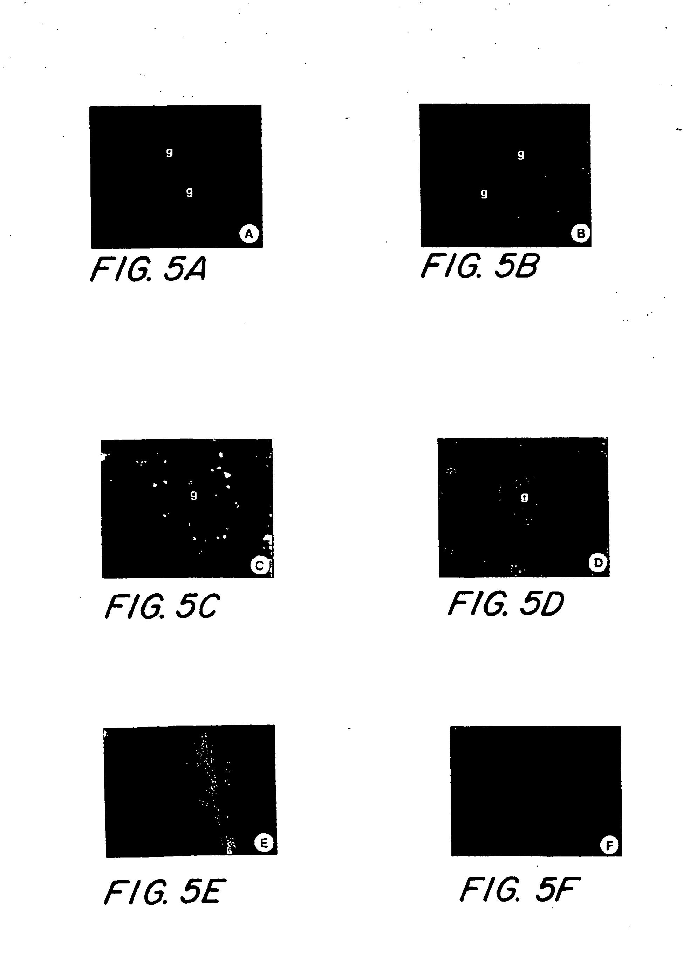

FIGURES 5A-5F show the immunohistochemical analysis of Fn deposition in the kidneys of normal and UG^{-/-} mice only in the absence of UG; (A) kidney section of a wild-type mouse that received a mixture of equimolar concentrations of Fn and UG intravenously; (B) UG^{+/+} mouse that received the same dose of Fn as in (A) but without UG; (C) apparently healthy, UG^{-/-} mouse receiving a mixture of Fn and UG; (D) UG^{-/-} mouse receiving Fn alone (same dose as in (C)), but without UG; (E) Fn-fibrillogenesis by cultured cells grown in medium supplemented with soluble hFn alone; (F) a cell culture identical to one in (E) which was fed with medium containing a mixture of equimolar concentrations of soluble hFn and UG (magnification 40X, g = glomerulus).

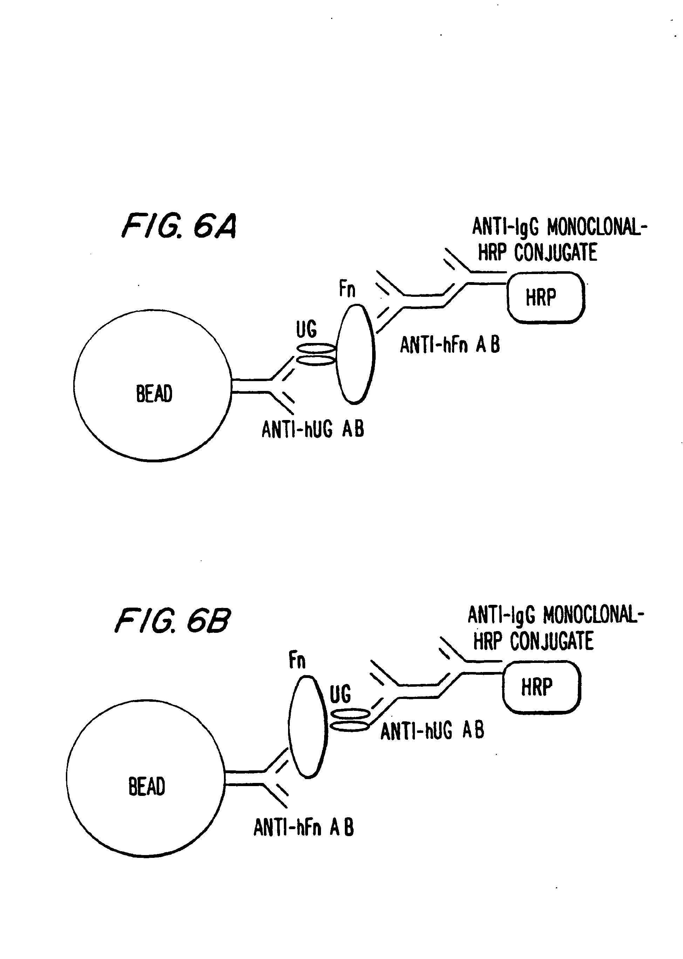

FIGURES 6A-6B show the format for a diagnostic assay to detect UG-Fn complexes in clinical samples.

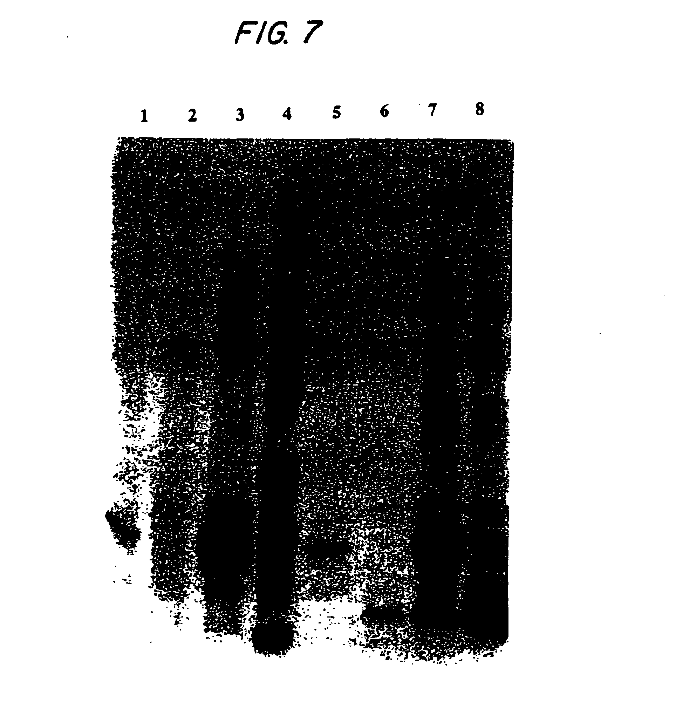

FIGURE 7 shows the passage of UG dimer through an 8.0 kDa MWCO dialysis membrane but not a 3.5 kDa MWCO dialysis membrane.

### DETAILED DESCRIPTION OF PREFERRED EMBODIMENTS

### rhUG

The rhUG of the invention has substantially the same amino acid sequence as that of the native human UG protein. An amino acid sequence having "substantially the same" amino acid sequence as that of the native human protein includes rhUG having at least 75% identity to the native human protein. In a preferred embodiment, rhUG has at least 85% identity, and in a most preferred embodiment, rhUG has at least 98% identity to the native UG.

Also included in the present invention is the use of fragments or derivatives of UG. A "fragment" of UG refers to a portion of the native hUG amino acid sequence having six or more contiguous amino acids of the native protein sequence. The term "derivative" refers to peptide analogs of UG, including one or more amino acid substitutions and/or the addition of one or more chemical moieties, e.g., acylating agents or sulfonating agent, with the proviso that the derivative retains the biological activity of the parent molecule.

Further, the UG used in the present invention is substantially pure. The term "substantially pure" refers to UG having a purity of about 75% to about 100%. In a preferred embodiment, UG has a purity of about 90% to about 100%, and in the most preferred embodiment, UG has a purity of at least 95%. Clinical Uses of UG

The invention provides, in another aspect, for treating or preventing an inflammatory or fibrotic condition in a mammal, which may be animal or human, with an effective amount of UG.

The following non-limiting list of conditions are representative examples of those associated with UG deficiencies, excessive PLA₂ activity, and fibronectin deposition.

**Table 2. Clinical Uses of Recombinant Uteroglobin (Grouped by UG Property)**

| UG Property | Condition(s) |
|---|---|
| UG deficiency | (1) Neonatal broncho-pulmonary dysplasia; |
| | (2) Complications of hemodialysis; |
| | (3) Bleomycin lung; |
| | (4) Chronic obstructive pulmonary disease; |
| | (5) Emphysema |
| Excessive PLA₂ activity | (1) Systemic inflammation; |
| (inflammation) | (2) Asthma; |
| | (3) Cystic fibrosis; |
| | (4) Ocular inflammation, including Autoimmune uveitis and corneal transplant surgery; |
| | (5) Conditions associated with obstetrics and gynecology, including premature labor and fertility (ex vivo) |
| Excessive PLA₂ activity (Immune modulation) | (1) Autoimmune diseases, including rheumatoid arthritis, Type I diabetes, Inflammatory bowel disease, and Crohn's disease; |
| | (2) Transplanted organ rejection |
| Fibronectin deposition | (1) Renal fibroses |
| | (2) Pulmonary fibroses, including idiopathic pulmonary fibrosis; |
| | (3) Vascular fibrosis |

Typical relationships between UG deficiency, PLA₂ activity and fibronectin aggregation and deposition in human inflammatory/fibrotic conditions are summarized below.

### Neonatal Broncho-Pulmonary Dysplasia (Neonatal BPD)

Neonatal BPD is characterized by severe inflammation and irreversible fibrosis of lung tissue in newborn infants, usually as a result of respiratory distress syndrome (RDS). However, this condition may also be caused by meconium aspiration syndrome or infection.

A deficiency of hUG has been implicated in this condition because the synthesis of pulmonary hUG may be coregulated with surfactant, which starts late in gestation. Thus, severely premature neonates may lack UG as well as surfactant. hUG deficiency may cause increased PLA₂ activity and Fn-related fibrosis, which are associated with the inflammation and fibrosis seen in neonatal BPD. Some infants do not respond to synthetic surfactant, which may be due to excess PLA₂ activity. Thus, UG may be used to treat neonatal BPD.

The preferred route of administration is direct instillation via the endotracheal or the systemic routes.

### Multiple Organ Failure (MOF)

Excessive PLA₂ activity has been implicated in MOF due to bacterial sepsis or trauma. This condition is characterized by a systemic inflammatory response, involving rapid, massive tissue damage and loss of organ function in the lungs, kidney, pancreas, intestines, and vasculature. Recent evidence points to the MOF trigger as elevated systemic soluble phospholipase A₂ activity, its direct lysis of tissue cell membranes, and hydrolysis of essential phospholipids, such as lung surfactants. Attempts to inhibit PLA₂ directly in clinical settings have been unsuccessful.

In MOF, the amount of endogenous UG is insufficient to counter the superactivation of PLA₂. Exogenously supplied UG can be used to combat MOF.

Remote organ failure (ROF) involves damage to organs other than the organ primarily affected by trauma or infection. Often remote organ failure involves more than one remote organ, resulting in multiple organ failure. For example, pancreatitis is an inflammation of the pancreas in response to alcohol intake, infection, or trauma, that may result in adult respiratory distress syndrome (ARDS), acute renal failure (ARF), and systemic shock. An episode of inflammatory bowel disease or peritonitis can result in ROF/MOF. ROF/MOF is associated with high levels of circulating, activated PLA₂. The systemic application of hUG could prevent ROF/MOF. The immediate injection of UG in patients with ROF/MOF, could reduce the severity or eliminate the PLA₂ mediated organ failure and shock.

### Pancreatitis

All forms of pancreatitis involve elevated Type I soluble PLA₂ activity, both systemic and local. Pancreatitis often results in pulmonary insufficiency or ARDS, characterized by elevated soluble PLA₂ activity in the lungs. Therefore, as an inhibitor of soluble Type I PLA₂s *in vivo*, UG is an excellent candidate for treatment of two forms of acute pancreatitis, and as a preventative measure of pulmonary insufficiency in all acute forms of pancreatitis.

The preferred route of administration is by the intravenous route.

### Inflammatory Bowel Disease

Inflammatory Bowel Disease (IBD), including ulcerative colitis, direticulitis, and Crohn's disease, is characterized by elevated local production and activity of Type II soluble PLA₂. Circulating soluble PLA₂ activity may also be elevated in IBD. IBD causes pulmonary insufficiency or ARDS in severe cases, as a result of elevated PLA₂ activity (which is similar to pancreatitis).

The rationale for the application of exogenous UG in IBD is identical to that of pancreatitis: to downregulate the inflammatory response by inhibiting PLA₂, Fn aggregation and/or deposition and to prevent remote organ involvement (lungs and kidneys).

The preferred route of administration is by the intravenous route in hospitalized patients.

### Bacterial Pneumonia

BAL fluids of patients who have survived bacterial pneumonia were shown to have 2-3X higher levels of UG than those who died. Bacterial infection of the lungs may overactivate the endogenous soluble PLA₂. UG may be administered to inhibit or control this effect.

The preferred route of administration is via the intratracheal route if the patient is intubated or intravenous if not.

### Complications of Dialysis

The major complication of dialysis is thromboses, i.e., spontaneously formed blood clots. These often plug the vascular access port, impairing treatment, as well as causing ischemic, sometimes life-threatening episodes, in the patient. A second problem with hemodialysis patients is inflammation and/or fibrosis of the proximal vein which returns the dialyzed blood to the patient's main circulation. Fibrosis of the proximal vein is usually detected as an increase in resistance, or pressure, against the return of the dialyzed blood. A third problem is fibrosis and closure of the vascular access site, or fistula. A fourth problem is accelerated atherosclerosis and a fifth is loss of residual renal function, most likely due to Fn deposition.

The possibility that endogenous UG is dialyzed away during the procedure provides an explanation for these problems. The selective removal of endogenous UG leaves circulating Fn free to aggregate, forming the foci for blood clot formation or to deposit on red blood cells, priming them for a clotting response by sticking to each other or to the vascular lumen. Transglutaminases (TGs) are enzymes responsible for building macromolecular lattices found in basement membranes, skin and blood clots. In the absence of free UG competing as a substrate for activated TGs, Fn and other components of blood clots are crosslinked.

Inflammation and fibrosis of both the proximal vein and the vascular access site, as well as accelerated atherosclerosis, may be explained by the deposition of Fn in the vascular lumen. Fibronectin deposition on the vascular endothelia promotes platelet and white blood cell adherence, both of which may be aggravated in the absence of PLA₂ inhibition. Vascular deposits of Fn may also promote local deposits of fat, cholesterol and protein found in atherosclerotic plaque. Fibronection is known to be a major component of atherosclerotic plaque, as well as renal glomercular deposits associated with nephropathy and loss of primary and residual renal function. Therefore, UG administration may reduce or eliminate these problems by reducing inflammation and fibronectin deposition.

The preferred route of administration of UG would be intravenous infusion before, during or after dialysis.

Alternatively, the loss of endogenous UG may be prevented by addition of UG to the dialysis buffer or precoating the dialysis membrane with UG or both.

### Organ Transplants

The term "organ" refers, for example, to solid organs, such as kidney, liver and heart, as well as bone marrow, cornea and skin.

There are two types of organ transplant rejection: acute and chronic. Acute rejection is an inflammatory process involving PLA₂ activity and infiltration by inflammatory cells that often destroys the graft.

Chronic rejection involves Fn-mediated fibrosis of the graft, including atherosclerosis confined to the graft. Thus, administration of UG may be used to treat or prevent both acute and chronic graft rejection.

The preferred route of administration is by injection.

Another aspect of organ transplantation is ischemia of the organ before removal from the donor, during transport and in the recipient, which contributes to acute rejection. Ischemia is known to result in elevated PLA₂ activity and tissue necrosis. Hence, UG could be used to prevent such ischemia. The preferred form of UG is as a perfusion liquid or storage buffer in which the ex vivo organ is preserved.

### Prevention of Type I Diabetes

Type 1 diabetes arises from the destruction of pancreatic tissue by an autoimmune response. The pancreas normally secretes soluble PLA₂s and hUG into the circulation. Necrotic lesions have been reported in the pancreas of the uteroglobin knockout (KO) mouse of the present invention (herein referred to as the "UG KO mouse").

In the absence of uteroglobin, the UG KO mouse exhibits similar pancreatic tissue destruction which could trigger an autoimmune response. Thus UG may be used to prevent or halt the slow progression of Type 1 diabetes. The preferred route of administration is by injection.

### Prevention and Treatment of Nephropathy

Renal Fn deposits and fibrosis in the UG KO mouse are similar to Fn deposits and fibrosis in human nephropathies. Thus, UG administration may prevent or slow the progression of nephropathy in patients at risk, such as Type II diabetes.

### Prevention and Treatment of Ocular Inflammation

Ocular inflammation, including uveitis, retinitis, and inflammation following surgery, is characterized by increased PLA₂ activity. Therefore, UG may be administered topically, intraocularly, or systemically to reduce ocular inflammation.

### Arteriosclerosis

Arteriosclerosis is a fibrotic thickening of blood vessels throughout the body. It is initiated and/or mediated by Fn deposition on the walls of the vasculature. Atherosclerosis is a form of arteriosclerosis involving cholesterol deposition, in addition to Fn deposition. Therefore, UG may be administered to prevent or reduce arteriosclerosis.

### Acute Renal Failure

Acute renal failure (ARF) is typically a consequence of remote organ inflammation, infection or direct trauma, which results in release and activation of soluble PLA₂ in the circulation. Damage to the kidneys during ARF can be quite severe, with acute tissue damage promoted by inflammation and may resolve into fibrosis of the kidney, leading to reduced kidney function in the long term. The anti-inflammatory and anti-fibrotic properties of UG are particularly relevant in the kidney as shown by the UG KO mouse.

The preferred route of administration is by injection or systemic administration.

Overall, the following non-limiting list of conditions are those associated with inhibition of PLA₂ and/or fibronectin deposition, each of which are candidates for treatment or prevention by the present invention:

| | |
|---|---|
| **Joint/Bone:** | rheumatoid arthritis; |
| **Autoimmune:** | rheumatoid arthritis, multiple sclerosis, Type 1 diabetes, uveitis, psoriasis, systemic lupus erythematosus (SLE), and Crohn's disease; |
| **Pancreas:** | pancreatitis; |
| **Peritoneum:** | peritonitis, appendicitis; |
| **Vascular/systemic:** | septic shock; collagen vascular disease, arteriosclerosis, atherosclerosis, anaphylactic shock, schistosomiasis, and trauma-induced shock; |
| **Renal:** | acute renal failure, bacterial infection of the kidneys, inflammation due to renal tumors, prevention of fibrosis resulting from chemotherapy or antibody therapy, prevention of diabetic nephropathy, prevention and/or treatment of idiopathic nephropathy; |
| **Liver:** | hepatitis, viral hepatitis, and cirrhosis; |
| **Bladder:** | cystitis, inflammation of the urethra, inflammation of the ureter, and bladder inflammation such as interstitial cystitis; |
| **Reproductive/female:** | vaginitis, inflamed cervix, pelvic inflammatory disease, inflammation of the ovary (salpingitis), endometriosis, vaginal candidiasis, and inflammation or fibrosis of the fallopian tubes; |
| **Reproductive/male:** | penile inflammation, prostate inflammation, inflammation of seminal tubules and vesicles, testicular inflammation, and inflammation of vas deferens, epididymis, and prostate gland; |
| **Ocular:** | uveitis, retinitis, trauma, burn damage due to chemical or smoke, ocular inflammation due to CMV retinitis, conjunctivitis (bacterial infection), viral infection, ocular inflammation due to infectious agent, ocular inflammation following ocular surgery, including cataract removal, laser surgery, corneal transplant, tumor removal, ocular inflammation due to retinoblastoma (tumor), ocular inflammation due to radiation exposure, inflammation due to allergic response; |
| **Heart:** | Endocarditis |
| **Lungs:** | bronchial asthma, ARDS, pneumonia, idiopathic pulmonary fibrosis, pulmonary fibrosis resulting from chemotherapy (bleomycin, methotrexate), pulmonary fibrosis resulting from exposure to environmental chemicals (asbestos, cleaning fluids, pollutants, e.g. dioxin and PCB's in automobile exhaust), smoke inhalation, pulmonary inflammation during recovery from drowning, and neonatal RDS; |
| **Gut:** | inflammatory bowel disease, colitis, Crohn's disease, direticulitis, neonatal necrotizing enterocolitis, inflammation due to an infectious agent, rotavirus, polio virus, HIV, stomach ulcers, gastro-esophageal reflux disease, and tonsillitis; |
| **Hemorrhoids;** | |
| **Transplants:** | administration following transplant surgery for any organ or tissue to control inflammation or fibrosis and rejections; |
| **Ears:** | Otitis media; and |
| **Skin:** | psoriasis, hives, allergic and dermatitis, scleroderma, contact dermatitis, chemical dermatitis (due to poison ivy, poison oak, and exposure to chemicals like PCB's, chlorine, ammonia, (cleaning agents, toxic agents). |

Moreover, UG may be administered either alone or in combination with other active agents or compositions typically used in the treatment or prevention of the above-identified disease conditions. Such active agents or compositions include, but are not limited to steroids, non-steroidal anti-inflammatories (NSAIDs), chemotherapeutics, analgesics, immunotherapeutics, antiviral agents, antifungal agents, vaccines, immunosuppressants, hematopoietic growth factors, hormones, cytokines, antibodies, antithrombotics, cardiovascular drugs, or fertility drugs. Also included are oral tolerance drugs, vitamins and minerals.

Moreover, UG may be administered as a carrier for the suppression of inflammation at the site of injection or administration of another therapeutic or prophylactic agent to suppress inflammation or an immune response caused by such agent. In this regard, UG would allow for administration of an effective amount of the active agent to the target indication.

UG may also be administered as a cosmetic composition for use in limiting fibrosis, scar or keloid formation resulting from wound healing, or during a dermal inflammatory response.

The present invention relates to the use of UG in the prevention or treatment of fibronectin associated conditions. With regard to prevention of a disease condition, "prevention" refers to preventing the development of disease in a susceptible or potentially susceptible population, or limiting its severity or progression, whereas the term "treatment" refers to the amelioration of a disease or pathological condition.

UG may be administered intravenously or, in the case of treatment of neonatal RDS/BPD and adult RDS, in the form of a liquid or semi-aerosol via the intratracheal tube. Other viable routes of administration include topical, ocular, dermal, transdermal, anal, systemic, intramuscular, slow release, oral, vaginal, intraduodenal, intraperitoneal, and intracolonic. Such compositions can be administered to a subject or patient in need of such administration in dosages and by techniques well known to those skilled in the medical, nutritional or veterinary arts taking into consideration such factors as the age, sex, weight, and condition of the particular subject or patient, and the route of administration. The compositions of the present invention may also be administered in a controlled-release formulation. The compositions can be co-administered or sequentially administered with other active agents, again, taking into consideration such factors as the age, sex, weight, and condition of the particular subject or patient, and, the route of administration.

Examples of compositions of the invention include edible compositions for oral administration such as solid or liquid formulations, for instance, capsules, tablets, pills, and the like liquid preparations for orifice, e.g., oral, nasal, anal, vaginal etc., formulation such as suspensions, syrups or elixirs; and, preparations for parenteral, subcutaneous, intradermal, intramuscular or intravenous administration (e.g., injectable administration), such as sterile suspensions or emulsions. However, the active ingredient in the compositions may complex with proteins such that when administered into the bloodstream, clotting may occur due to precipitation of blood proteins; and, the skilled artisan should take this into account.

In such compositions UG may be in admixture with a suitable carrier, diluent, or excipient such as sterile water, physiological saline, glucose, DMSO, ethanol, or the like. UG can be provided in lyophilized form for reconstituting, for instance, in isotonic aqueous, saline, glucose, or DMSO buffer. In certain saline solutions, some precipitation of rhUG has been observed; and this observation may be employed as a means to isolate inventive compounds, e.g., by a "salting out" procedure.

Further, the invention also comprehends a kit wherein UG is provided. The kit can include a separate container containing a suitable carrier, diluent or excipient. The kit can include an additional agent which reduces or alleviates the ill effects of the above-identified conditions for co- or sequential-administration. The additional agent(s) can be provided in separate container(s) or in admixture with UG. Additionally, the kit can include instructions for mixing or combining ingredients and/or administration.

The invention also contemplates treating or preventing a fibrotic condition characterized by a deficiency of endogenous functional UG, which comprises administering to a patient in need of such treatment a compensating amount of UG. The term "compensating amount" means an amount of UG required to bring the local pulmonary or systemic concentration of total UG (endogenous functional UG and exogenous UG) to within its normal range. More specifically, the normal range for local pulmonary concentration of endogenous UG is about > 50 micrograms UG/milligram albumin or > 50 micrograms/liter. The normal range for serum UG concentration is > 15 micrograms/liter.

The compositions of the invention comprise native and/or recombinant hUG in an amount effective to achieve the intended purpose, namely increased plasma or tissue levels of UG to produce the desired effect of selective inhibition of PLA₂s and reduced inflammation and/or binding of fibronectin to mitigate its aggregation and/or deposition in fibrotic conditions. The compositions comprise an effective amount of substantially pure native and/or recombinant human UG, in association with a pharmaceutically acceptable carrier or diluent.

In a further aspect, the compositions of the invention comprise an effective amount of native and/or recombinant hUG and lung surfactant, in association with a pharmaceutically acceptable carrier or diluent. Local intratracheal administration of UG to the lungs, sufficient for inhibition of PLA₂ activity and/or fibronectin deposition requires quantities of substantially pure UG in the range of 0.2 µg/kg to 500 mg/kg of protein in single or multiple dosages. The UG is usually administered in an amount of a single bolus of 20 ng/kg to 500 mg/kg, in single or multiple doses, or as a continuous infusion of up to 10 grams.

Native and/or recombinant hUG may also be administered in conjunction with artificial lung surfactant, such as Survanta, via the intratracheal route. UG and Survanta (5 mls/kg) are co-administered and UG does not bind surfactant, preventing it from functioning therapeutically. The lung surfactant is generally present in the composition an amount of about 10-90 % by weight, more usually about 20-80 % by weight. The surfactant, by virtue of its very low surface tension spreads out over the internal surface of the lungs, carrying the UG with it. Systemic administration of UG via intravenous injection, sufficient for inhibition of PLA₂ activity and/or fibronectin deposition, requires quantities of substantially pure native and/or recombinant hUG in the range of 0.5 µg to a continuous infusion of several grams of protein over an extended period of time (days).

Suitable formulations for injection and semi-aerosol intratracheal delivery include aqueous solutions of native and/or recombinant hUG optionally with viscosity modifiers and stabilizers.

The term "PLA₂ inhibiting effective amount" as used herein means the amount of UG which inhibits PLA₂ activity and which reduces or alleviates inflammation in the tissue or body of the patient. The term "fibronectin binding effective amount" means that amount of UG which binds fibronectin to reduce aggregation and/or deposition thereof, and prevent or reduce fibrillogenesis or fibrosis. The term "anti-inflammatory amount" as used herein means the amount which reduces or alleviates inflammation in the tissue or body. Typically, the amount of UG administered to adults for the treatment of inflammatory and fibrotic conditions will be single boluses of 0.2 µg/kg to 500 mg/kg or up to several grams administered over an extended period of time. For neonates, in the treatment of neonatal RDS, the range will typically be 50 nanograms/kg to 100 mg/kg in single boluses or up to 10 grams administered continuously over an extended period of time. Effective and safe rates of continuous infusion are between 50 ng/kg/hour to 500 mg/kg/hour.

### EXAMPLES

The invention will now be further described with reference to the following non-limiting examples. Parts and percentages are by weight unless otherwise stated.

### Example 1: In Vivo Experiments

Recombinant human UG was obtained by the method of Mantile *et al*. (1993).

One male and one female of the species *P. cyanocephalus*, weighing approximately 400 grams each were delivered by C-section at 142 days of gestation. This is an established model of RDS (Coalson, J.J., et al. Baboon Model of BPD. II: Pathologic features. Exp. Mol. Pathol. 37: 355-350 (1982)).

After delivery, the infants were anesthetized with ketamine (10 mg/kg) and intubated with a 2.5 mm diameter endotracheal tube. Blood gases and pressure were monitored via an arterial line placed by percutaneous injection into the radial artery. A deep venous line was placed percutaneously into the saphenous vein through which fluids, antibiotics, and drugs were administered. Animals were maintained on servo-controlled infrared warmers and ventilated with a standard time-cycled, pressure-regulated ventilator with humidifiers maintained at 36-37°C. Initial setting were FiO₂1.0, rate 40/min., I/E ratio 1:1.5, positive end expiratory pressure (PHEP) at 4 cm H₂O, and peak inspiratory pressure (PIP) as required for adequate chest excursion. FiO₂ was kept at 1.0 and PIP was regulated to maintain PaCO₂ at 40±10 torr. Blood gases, hematocrit, electrolytes, prothrombin time, partial thromboplastin time and dextrostix were monitored hourly. - Blood drawn for studies was replaced volumetrically with heparinized adult baboon blood. Intravenous fluids were administered with electrolytes at 10 cc/kg/hr and were increased as needed when heart rate exceeded 180 beats/min. Sodium bicarbonate (2 meq/kg) was administered when the base deficit exceeded -10. Ampicillin (50 mg/kg/day in two divided doses) and Gentamicin (5 mg/kg/day in two divided doses) was given continuously for the duration of the experiment.

One animal received surfactant plus PBS (treatment no. 1), and the second animal (treatment no. 2) received surfactant plus two doses of 1 mg/kg of rhUG. Both surfactant and rhUG were administered directly to the lungs through the endotracheal tube. The surfactant used was Survanta (Ross Labs), a surfactant preparation derived from bovine lung tissue, containing surfactant apoproteins B and C in addition to phospholipids. The first dose of rhUG was given with the surfactant and the second administered four hours after the first. The animals were monitored for arterial blood gases, electrolytes and EKG. They were sacrificed 50 hours after the initiation of surfactant therapy. The lungs were lavaged at 24 and 48 hours with PBS containing protease inhibitors (PMSF, 10 µg/ml leupeptin, 10 µg/ml of pepstatin and bacitracin). They were frozen at -80°C until assayed for PLA₂ activity. Total proteins were determined by Bradford method (BioRad). The PLA₂ activity in the lung lavages were measured according to Levin et al. (1986; *supra*) and are presented in the following Table.

**Table 3. Results of In Vivo Testing of UG**

| Treatment # | Time | Lung lavage PLA₂ activity (ccpm/10 µg protein |
|---|---|---|
| 1 | 24 hr | 3030 |
| | 48 hr | 2607 |
| 2 | 24 hr | 1739 |
| | 48 hr | 996 |

The data given above are the mean of two determinations. The results show that endotracheal administration of rhUG inhibits PLA₂ *in vivo.* The animals which received surfactant and rhUG had an appreciably lower PLA₂ activity in their lung lavage fluid compared with the animals that received surfactant without rhUG. The data confirm that administration of rhUG in conjunction with surfactant is beneficial in protecting surfactant phospholipids.

### Example 2: Inhibition of Hydrolysis of Artificial Surfactant by Soluble PLA₂ in vitro

RhUG inhibits hydrolysis of artificial surfactant by soluble PLA₂s *in vitro.* Survanta is an artificial surfactant derived from bovine lung and is used to treat pre-term neonates with RDS and adults with RDS (ARDS). Hydrolysis of Survanta by a Group I soluble PLA₂, i.e. porcine, pancreatic PLA₂ (Boehringer Mannheim) is characterized by its ability to compete as a substrate with a fluorescent phosphatidylcholine substrate (Cayman Chemicals), generating arachidonic acid as a product.

Survanta is a substrate for *in vitro* degradation by Group I soluble PLA₂s. Survanta is rapidly degraded *in vitro* by PLA₂s found in the extracellular fluids of a human lung. RhUG inhibits degradation of Survanta *in vitro.*

### Example 3: Construction of UG knockout mouse

A transgenic UG KO mouse was created for the purpose of determining the role of uteroglobin in mammalian physiology, as well as to generate a model for UG as a therapeutic in several inflammatory clinical conditions. The first step was to construct an appropriate DNA vector with which to target and interrupt the endogenous murine uteroglobin gene. The 3.2 kb BamHI-EcoRI DNA fragment containing exon 3 and flanking sequences of the uteroglobin gene from the 129/SVJ mouse strain (Ray, 1993) were subcloned into the corresponding sites of the pPNW vector as described in Lei et al (1996). A 0.9 kb fragment containing part of exon 2 and its upstream sequence was amplified by PCR (with primers Primer-L (from Intron 1): 5'-TTC CAA GGC AGA ACA TTT GAG AC-3'; Primer-R (from Exon 2): 5'-TCT GAG CCA GGG TTG AAA GG C-3') with NotI and XhoI restriction sites engineered into the termini for directional subcloning into the gene targeting vector. In this construct, 79 bp of Exon 2 encoding 27 amino acids were deleted. The PCR fragment was placed upstream of the gene encoding neomycin resistance in pPNW, generating the gene targeting vector, pPNWUG. The vector is shown in Figure 2A, in which the PGK-neo cassette interrupts the uteroglobin gene, disrupting the protein coding sequence.

The pPNWUG gene targeting vector was linearized with NotI and electroporated into ES R₁ cells according to Nagy, A., et al. PNAS 90:8424 (1993). Gancyclovir and G-418 selection of the electroporated cells yielded 156 clones. Southern (DNA) blot analysis identified a 5.1 kb HindIII fragment of the wild-type uteroglobin allele and an additional 8.2 kb HindIII fragment resulting from homologous recombination in three out of the 156 clones, shown in Figure 2B. These ES R1 clones were injected into C57BL/6 blastocysts according to Capecchi, Science 244: 1288 (1989). Two different lines of mice, descended from different chimeric founders, were generated. Heterozygous offspring (UG^{+/-}) carrying the targeted uteroglobin gene locus were mated and the genotypes of the progeny were analyzed by PCR shown in Figure 2C, as well as Southern blot, shown in Figure 2D.

### Example 4: Verification of UG gene knockout and absence of murine UG (mUG) protein

In order to verify that the homozygous knockout mice (UG^{-/-}) did not possess any detectable mUG, the uteroglobin gene-targeted mice were tested for expression ofm UG-mRNA and mUG protein in several organs including the lungs. An experimental protocol was approved by the institutional animal care and use committee. Total RNAs were isolated from different organs of UG^{+/+}, UG^{+/-}, and UG^{-/-} mice. The reverse transcribed-polymerase chain reaction (RT-PCR) was used to detect mUG-mRNA. Target molecules were reverse transcribed using a mUG-specific primer, mPr (5'- ATC TTG CTT ACA CAG AGG ACT TG-3'), and the cDNA generated was amplified using PCR primers mPr and mPl (5'-ATC GCC ATC ACA ATC ACT GT-3'). The PCR product was hybridized with an oligonucleotide probe, mPp (5'-ATC AGA GTC TGG TTA TGT GGC ATC C-3') derived from exon-2 of the UG gene sequence. The primers and the probe used in mouse GAPDH RT-PCR are as follows: mGAPDH-r (5'-GGC ATC GAA GGT GGA AGA GT-3'); mGAPDH-1 (5'-ATG GCC TTC CGT GTT CCT AC-3'); mGAPDH-p (5'-GAA GGT GGT GAA GCA GGC ATC TGA GG-3'). Figure 2E shows that mUG-mRNA was detected in the lungs of UG^{+/+}, and UG^{+/-}, but not UG^{-/-} mice. Similar data (not shown) show that mUG-mRNA is not present in either the prostate or uteri of UG^{-/-} mice, but is present in the mice with an intact uteroglobin gene.

Immunoprecipitation and Western blot analyses of mUG protein in the lungs yielded similar corroborative results, shown in Figure 2F. Tissue lysates from the kidneys, liver, and the lungs of the UG^{+/+} and UG^{-/-} mice were prepared by homogenizing in a buffer (10 mM Tris-HCl, pH 7.5, 1% Triton X-100, 0.2% deoxycholate, 150 mM NaCl, 5 mM EDTA) containing 2 mM phenylmethylsulfonyl fluoride and 20 µg/mL each of aprotinin, leupeptin, and pepstatin A. The homogenates were centrifuged at 17,500 x g for 30 min at 4°C and immunoprecipitated as described (E. Harlow and D. Lane, Antibodies; a laboratory manual, 1st Ed. Cold Spring Harbor Laboratory Press, Cold Spring Harbor, NY, 1988) by incubating tissue lysates or plasma proteins (1 mg/mL) with rabbit antibody against murine Fn (1:100 dilution). Co-immunoprecipitation of purified murine Fn (mFn) and rhUG (Mantile, G, et al., J. Biol. Chem. 267: 20343 (1993)) was performed by incubating equimolar concentrations of mFn with rhUG in the presence of 10% glycerol, 50 mM Tris-HCl, pH 7.5, 250 mM NaCl, 4.3 mM sodium phosphate at 4°C for 1 hr., followed by adding anti-mFn antibody (1:100 dilution). Equal amounts of extracted tissue proteins (30 µg) or immunoprecipitates were resolved either on 4-20% or 6% SDS-polyacrylamide gels under reducing conditions, followed by Western blotting with rabbit antibodies against either murine Fn (1:2000 dilution) or UG (1:2000 dilution). No mUG was detected in tissues or fluids from the UG^{-/-} mice, while tissues from UG^{+/+} and UG^{+/-} mice did contain the mUG protein.

Finally, histopathological analyses of the lungs of UG^{-/-}, only, lacked mUG-specific immunostaining in bronchiolar epithelial cells. Lung tissues from UG^{-/-}, UG^{+/-} and UG^{+/+} mice were fixed in Bouin's fluid or in 10% neutral buffered formalin fixatives, embedded in paraffin and sectioned at 4-6 microns. They were stained with hemotoxylin and eosin (H & E). Selected tissues were stained by Masson's trichrome method for collagen detection, PTAH for fibrin, or Congo Red for amyloid protein. For immunohistochemical detection of mUG and mFn, the Vectastain rabbit Elite ABC kit (Vector Laboratories) was used. The rabbit antibody (CytImmune) to mUG was raised by using a synthetic peptide (Peptide Technologies, Inc.) corresponding to mUG amino acid sequence (Lys28 to Thr49, specifically KPFNPGSDLQNAGTQLKRLVDT). The rabbit antibody to mFn (GIBCO BRL) was used at a dilution of 1:1000, and the antibody to mUG was used at 1:500.

These three sets of results confirm that the homozygous uteroglobin knockout mouse, UG^{-/-}, lacks mUG protein, or any detectable piece of the protein.

### Example 5: Phenotype of uteroglobin knockout mouse

Of the 179 mice born to crosses of UG^{+/-} mice, 46 (26%) were of the +/+, 90 (50%) of the +/- and 43 (24%) of the UG^{-/-} genotype, indicating that the disrupted mUG locus is inherited in a Mendelian fashion and that UG^{+/+}, UG^{+/-}, and UG^{-/-} mice were equally viable at birth. However, UG^{-/-} mice exhibited a novel phenotype in which they developed a progressive illness characterized by cachexia, heavy proteinuria, and hypocalcemia associated with profound weight loss. Proteinuria is a condition in which abnormally high levels of albumin and other serum proteins are excreted in the urine. It is indicative of glomerular dysfunction and renal failure. Histopathological examination of the kidneys of affected animals (as described above for lungs) revealed the fulminant renal glomerular disease shown in Figure 3. Compared with the glomeruli of the UG^{+/+} mice, those of UG^{-/-} mice were hypocellular and had massive eosinophilic proteinaceous deposits. The time course of the fatal renal disease in UG^{-/-} mice was either early onset (4-5 week period) or late onset (10 month period). Those UG^{-/-} mice that initially appeared healthy at 4 weeks of age had focal glomerular deposits at two months of age. At about 10 months, these mice had extreme cachexia similar to that of the mice dying of early onset disease. Heterozygotes had a milder form of the renal disease observed in UG^{-/-} mice. Histopathology of the kidneys of mice with late onset disease showed not only severe glomerularopathy as in the early onset disease, but also had marked fibrosis of the renal parenchyma and tubular hyperplasia (see Figure 3). Although the predominant pathology in the UG^{-/-} mice was found in the kidneys, histopathological studies also uncovered occasional focal areas of necrosis in the pancreas which appeared to be vascular oriented. Moreover, focal areas in the thymus and in the spleen structures suggestive of apoptotic bodies were also found. Interestingly, the pancreas expresses the mUG gene, and this organ is also a rich source of group-I extracellular PLA₂; since this is primarily a digestive enzyme, its activation may cause tissue injury.

Because uteroglobin proteins have has been reported to have immunomodulatory and anti-inflammatory properties and because reactive amyloidosis is known to occur in response to inflammation, it was likely that the glomerular deposits in the mUG-null mice were amyloid proteins. Reactive amyloidosis is characterized by the deposition of amyloid protein and immune complexes. The identity of the renal deposits in the UG^{-/-} mice was established by immunohistochemistry of kidney sections. Kidney sections from UG^{-/-} and UG^{+/+} mice were stained with Congo red and examined under the polarized light. Amyloid proteins yield a positive birefringence in this test; however, the glomeruli of UG^{-/-} mice were clearly negative. Immunofluorescence studies for the presence of IgA, IgG or IgM-immunocomplexes in the glomeruli of UG^{-/-} mice and immunohistochemical analyses for the presence of major amyloid proteins were also negative. Thus, the glomerular deposits of UG^{-/-} mice contained neither amyloid proteins nor immunocomplexes, and therefore, do not appear to be the result of an inflammatory response.

### Example 6: Detection of Fn and Collagen in UG^{-/-} kidneys

The kidney deposits of UG^{-/-} mice were examined by transmission electron microscopy to elucidate their structure and morphology. A kidney from a UG^{-/-} mouse, with glomerular lesion, was fixed in formalin and embedded in epoxy resin. Thin sections were stained with uranyl acetate and lead citrate for examination under the electron microscope. Photomicrographs were taken either at 6000X or at 60,000X. The deposits contained primarily two types of fibrillar structures: one type of long and striated fibrils which are relatively infrequent, the other short and diffuse which are more abundant (Figures 3E and 3F). Because ECM proteins, such as collagen and fibronectin, produce similar fibrillar structures, the glomerular deposits in UG^{-/-} mice may contain these proteins.

The glomerular deposits were next analyzed by immunofluorescence using anti-mFn antibody. Formalin-fixed tissue sections were used for immunofluorescence using a rabbit anti-mFn and FITC-conjugated goat anti-rabbit IgG. Similarly, immunofluorescence studies using antibodies specific for mFn, collagen I and III, vitronectin, laminin and osteopontin were also done. Epifluorescence was photographed using a Zeiss Axiophot microscope. Fn-specific immunofluorescence in the renal glomeruli of wild-type mice was virtually undetectable (Figure 3G), that in the glomeruli of UG^{-/-} littermates was intense (Figure 3H). When Masson's trichrome staining was used, the glomeruli of UG^{+/+} mice were negative (Figure 3I) and those of UG^{-/-} (Figure 3J) mice were positive, suggesting the presence of collagen in the glomerular deposits. Immunofluorescence, using collagen I and collagen III-specific antibodies confirmed these results. Because Fn is known to interact with other extracellular matrix (ECM) proteins, we also tested for the presence of laminin, vitronectin and osteopontin in the glomeruli of UG^{+/+} and UG^{-/-} mice by immunohistochemistry, the results of which were negative.

### Example 7: Kidneys of UG^{-/-} mice do not overproduce Fn

In order to determine whether excessive production of Fn may account for its deposition in the renal glomeruli, we assessed the relative amount of Fn-mRNA in the kidneys, lungs, and the liver of UG^{-/-} and UG^{+/+} mice by RT-PCR and densitometry. The results indicate that relative amounts of Fn-mRNA were essentially identical in both UG^{+/+} and UG^{-/-} animals. Thus, over-production of Fn-mRNA was not a likely cause of Fn-deposition in the glomeruli of UG^{-/-} mice. We then compared the Fn-protein in the plasma, kidneys, and the liver of UG^{-/-} and UG^{+/+} mice by SDS-PAGE under reducing conditions, and Western blotting. In the plasma, kidneys and the liver of wild-type mice only 220-kD Fn species could be detected; however, whereas the plasma and the liver lysate of UG^{-/-} mice had the 220-kD Fn band, the kidney lysates contained another distinct, covalently linked, multimeric Fn-band (Figure 4A).

### Example 8: Elevated serum PLA₂ activity in UG^{-/-} mice

Based upon current concepts, critical initial steps in Fn matrix-assembly and fibrilogenesis, at least on the cell surface, are thought to involve integrin activation and Fn self-aggregation. Because UG is a potent inhibitor of soluble phospholipase A₂ (sPLA₂), a key enzyme in the inflammatory pathway, the lack of mUG in UG^{-/-} mice may contribute to the development of glomerulonephritis, an inflammatory renal disease. Thus, we measured PLA₂ activity in the serum of age, sex and weight-matched UG^{+/+} (n=3) and UG^{-/-} mice (n=3). The animals were sacrificed and serum PLA₂ activities of each sample were measured in triplicate using a PLA₂-assay kit (Caymen Chemical) according to the instructions of the manufacturer. Protein concentrations in the sera were determined by Bradford assay (Bio Rad) and specific activities of PLA₂ were calculated. The specific activities (µmol/min/mg protein) of serum PLA₂ of UG^{-/-} mice [36+ 3.3 (SEM)] were significantly higher (p < 0.05) than those of UG^{+/+} mice [18+2.8 (SEM)]. These results raised the possibility that higher PLA₂ activity may lead to increased lysophosphatidic acid (LPA) production and consequently promote integrin activation and Fn-self aggregation in UG^{-/-} mice.

### Example 9: Interaction of uteroglobin and fibronectin in vitro

To further understand how uteroglobin may prevent Fn self assembly, the ability of rhUG to disrupt mFn-Fn interaction *in vitro* was determined. Equimolar concentrations of rhUG and mFn were incubated to allow any protein binding or other interactions, then immunoprecipitated with anti-Fn-antibody, and the immunoprecipitates were resolved by SDS-PAGE under reducing conditions. Western blotting, as previously described, with either mFn or mUG antibody detected each protein, respectively. The results show that fibronectin co-immunoprecipitated with rhUG (Figure 4B). To confirm these results, the ¹²⁵I-rhUG was incubated with mFn and the complexes resolved by electrophoresis, using a 6% polyacrylamide gel under non-denaturing and non-reducing conditions (Figure 4C). Detection of a Fn-UG heteromer in the autoradiogram (lane 2) showed that soluble Fn interacts with UG *in vitro*. To ascertain whether Fn-UG heteromerization takes place in vivo, plasma of UG^{+/+} and UG^{-/-} mice was immunoprecipitated with an anti-mFn antibody that does not crossreact with rhUG (Figure 4D). Anti-mFn antibody co-precipitated both mFn and rhUG from the plasma of UG^{+/+}, but not from UG^{-/-} mice, suggesting that Fn-UG heteromers are present in the plasma of UG^{+/+} mice. Therefore, the Fn-UG complex is not simply an artifact formed *in vitro* but occurs naturally in the serum.

To determine the specificity and affinity of UG binding to Fn, we incubated ¹²⁵I-Fn with unlabeled Fn in the presence and absence of UG. Any complexes were affinity-crosslinked with disuccinimidyl suberate (DSS). Using 24-well plates coated with human Fn (hFn) (Collaborative Biomedical Products), 3 µl of ¹²⁵I-Fn (Sp. Act. 6 mCi/mg: ICN Biomedicals) was incubated in the absence and presence of either UG or Fn (10⁻¹²-10⁻⁶ M) in 500 µl HBSS at room temperature for 2 hr. SDS-PAGE and Western blotting of all Fn with UG antibody failed to detect any UG contamination. The radiolabeled complex was washed twice with PBS, solubilized in 1 N NaOH, neutralized with 1 N HCl, and radioactivity was measured by a gamma counter. In a separate experiment ¹²⁵I-hFn (3 µl) was incubated with 20 µl (1 mg/ml) of mouse Fn in 40 µl of HBSS, pH 7.6 in the absence or presence of increasing concentrations of reduced rhUG (5-500 µg) at room temperature for 2 hours. The samples were crosslinked with 0.20 mM DSS at room temperature for 20 min., boiled in SDS-sample buffer for 5 min., electrophoresed on 4-20% SDS-polyacrylamide gel and autoradiographed. In the absence of UG, ¹²⁵I-Fn formed a high molecular weight, radioactive complex with unlabeled Fn, but in the presence of UG the formation of Fn-Fn aggregates was inhibited in a manner dependent upon the UG concentration (Figure 4E).

To determine whether there is any difference between the binding affinities of Fn for UG and that of Fn for itself binding experiments were performed in which ¹²⁵I-Fn was incubated with unlabeled Fn and immobilized on multiwell plates together with varying concentrations of UG. In separate experiments, binding studies of ¹²⁵I-Fn with unlabeled, immobilized Fn using various concentrations of unlabeled soluble Fn, were also done. The Scatchard analyses of the data from both types of binding experiments yielded straight lines with dissociation constants (kds) of 13 nM for UG binding to Fn and 176 nM for Fn binding to itself. These results suggest that, due to a relatively higher binding-affinity of UG for Fn, UG effectively counteracts Fn self-aggregation. Affinity-crosslinking experiments in which radio-iodinated (¹²⁵I)-collagen I was incubated with unlabeled Fn in the absence or presence of UG, were also done as described above for Fn. Fifteen µl of either denatured or non-denatured ¹²⁵I-collagen I (Sp. Act. 65.4 mCi/mg) were incubated with Fn in presence or absence of reduced UG (250 µg), affinity crosslinked, electrophoresed and autoradiographed. The results indicate that UG counteracts the formation of high molecular weight ¹²⁵I-collagen-Fn aggregates (Figure 4F).

### Example 10: In vivo inhibition of glomerular hFn deposition by rhUG

To test whether rhUG protects the renal glomeruli from Fn accumulation, soluble human Fn (hFn) alone, or hFn mixed with equimolar concentrations of rhUG, was administered intravenously to UG^{+/+} and to apparently healthy UG^{-/-} littermates.

Human Fn (500 µg/150 µl PBS) was administered in the tail vein of two-month old, approximately 22 g, UG^{+/+} and apparently healthy, UG^{-/-} mice. Similarly, the control mice were injected with a mixture of 500 µg of hFn either with equimolar concentrations of rhUG or albumin in 150 µl PBS. Twenty-four hours after the last injection, the mice were sacrificed and various organs were fixed in buffered formalin. The histological sections of the kidneys and other organs were examined by immunofluorescence with a monospecific anti-hFn antibody (GIBCO BRL; clone 1) and FITC conjugated rabbit anti-mouse IgG (Cappel). In a separate experiment, UG^{+/+} mice were injected with 1 mg of hFn alone in 150 µl PBS daily for 3 consecutive days.

The rationale for injecting human Fn was to be able to discriminate between endogenous murine Fn and the administered hFn. The method of intravenous administration and immunohistochemical detection of hFn in various tissues have been described.

Human Fn immunofluorescence in the glomeruli of wild-type UG^{+/+} mice injected with either a mixture of hFn and rhUG (1:1 molar ratio) or with hFn alone was similar (Figures 5A and 5B). However, the UG^{-/-} mice injected with a mixture of hFn and UG showed little hFn-specific immunofluorescence in the glomeruli (Figure 5C), while those receiving Fn alone exhibited higher intensity immunofluorescence (Figure 5D). Administration of a mixture of hFn and BSA, as a control, yielded no protective effect.

To determine whether this UG protective effect could be overcome by injecting larger quantities of Fn in UG^{+/+} mice, we injected 1 mg of hFn per animal daily for three consecutive days. Although intravenous administration of hFn to UG^{+/+} mice at lower doses (500 µg/animal) was not effective in causing any appreciable glomerular deposition (Figure 5A), the administration of higher doses (3 mg/animal) led to a significant accumulation. Thus, UG prevents glomerular Fn-deposition, and UG^{+/+} as opposed to UG^{-/-} mice have a higher threshold for the accumulation of soluble Fn, due to the presence of endogenous UG.

### Example 11: Inhibition of fibrilogenesis and Fn matrix assembly by rhUG in tissue culture cells

To determine whether UG prevents Fn-fibrilogenesis and matrix assembly in a typical *in vitro* tissue culture assay, mouse embryonic fibroblasts were cultured in medium containing either soluble hFn alone or a mixture of equimolar concentrations of hFn and rhUG. Fn matrix assembly and fibrilogenesis in cultured cells (CRL6336, ATCC) were determined as described. The level of fibrilogenesis seen in the cells of cultures treated with hFn alone was much higher (Figure 5E) compared to those which received a mixture of hFn and rhUG (Figure 5F).

### Example 12: Detection of UG-Fn complexes in clinical samples

Detection of UG-Fn complexes in clinical samples of bodily fluids such as serum, BAL fluids, and sputum is important in determining the role of this complex in human disease. A solution phase diagnostic assay for the detection of UG-Fn complexes is developed and the assay format is shown in Figure 6. The capture antibody, covalently linked to a solid support, is a monospecific rabbit polyclonal raised against the human protein. The solid support may be a bead, such as a magnetic bead, a tube, or an ELISA plate. The solid support affords the flexibility of performing wash steps after each binding reaction in order to obtain more consistent results with a variety of sample types. The detection antibody is specific for Fn, and available from a number of commercial sources. An anti-IgG antibody, conjugated to an enzyme such as horse radish peroxidase (HRP), is then used to detect the anti-Fn IgG at the end of the molecular chain in a standard enzymatic reaction in which the enzyme substrate is converted to a chromogenic or fluorogenic compound that is quantitated with a spectrophotometer or fluorimeter (Amersham). The detection limit for this assay is 500 µg of UG-Fn complex per ml of sample fluid.

### Example 13: Uteroglobin Deficiencies

A transient but acute deficiency of hUG is created by the blood-cleansing technique known as clinical dialysis, including hemodialysis, peritoneal dialysis and continuous dialysis (CRRT). All forms of clinical dialysis involve the use of a semipermeable membrane to filter toxic bodily waste products, including chemical metabolites such as urea, and small proteins such as beta2-microglobulin, out of the blood.

UG is an extremely compact protein, known for its anomolous migration in SDS-PAGE, corresponding to a molecular weight of approximately 10-13 kDa, despite its true molecular weight of 15.7 kDa. Therefore, the UG dimer was expected to behave as a 10-13 kDa protein in dialysis experiments. Surprisingly, it was found that the dimer is so compact that it passed through an 8.0 kDa MWCO dialysis membrane. UG also passed through a 14.0 kDa MWCO dialysis membrane.

The composition of the dialysis membranes used in these examples are similar, if not identical, to the composition of the majority of membranes manufactured and used for clinical dialysis. They consist of regenerated cellulose or cellulose acetate.

For this experiment, 1.0 ml aliquots of two partially purified (one > 90% pure and one approximately 70% pure) rhUG cell lysates, with no buffer additives, were dialyzed against 1000 mls of unbuffered 50 mM ammonium acetate, using three sizes of dialysis tubing: 3.5 kDa, 8.0 kDa, and 14.0 kDa (Spectra/Por; Thomas Scientific). There were four changes of buffer for each sample over a 48 hour time period, all done at room temperature (about 25-27°C). The appearance of each dialysis sample changed from a clear yellow to a clear, colorless liquid. Dialysis tubing was checked for leaks at the beginning and end of the process by brief application of pressure directly to the tubing (squeezing) and observation of any leaks, of which there were none. Tubing was double clamped at either end to further insure against leaks.

Figure 7 shows the SDS-PAGE analysis of these results. The 90% pure predialysis sample is shown in lane 7 and 8 next to the three post-dialysis samples in lanes 1, 2, and 3. The UG dimer is no longer present in the lanes representing the samples dialysed with 8.0 kDa MWCO membranes. These results were later confirmed with different batches of partially purified UG preparations.

### REFERENCES

1. Levin, S.W. et al., Life Sci. 38: 1813-1819 (1986);
2. Singh G. et al., Biochem. Biophys. Acta. 1039: 348-355(1990);
3. Mantile, G. et al., J. Biol. Chem 268: 20343-20351 (1993);
4. Singh, G. et al., J. Histochem. Cytochem. 36: 73-80 (1987);
5. Bernard, A. et al., Clin. Chem. 38: 434-435 (1992);
6. Dhanireddy, R. et al., Pediatric Res. 23: 463A (1988);
7. Dhanireddy, R. et al., Pediatric Res. 33: 323A (1993);
8. Piomelli, D. , Op. In Cell Biol. 5: 274-280(1993);
9. Krishnan, R.S. et al., Science 158: 490-492 (1967);
10. Beier, H. Verhandl Deut. Zool. Ges. Heidelberg (1968);
11. Umland, T.C. et al., Nature Stuct. Biol. 1: 538-545 (1994);
12. Hard, T. et al., Nature. Struct. Biol. 2: 938-989 (1995);
13. Umland, T.C. et al., Nature Struct. Biol. 2: 919-922(1995);
14. Stripp, B. R. et al., Am. T. Physio. 271 (Lung Cell. Mol. Physiol. 15): L656-L664 (1996);
15. Lesur, O. et al., Am. T. Respir. Crit. Care Med. 152: 290-297 (1995);
16. Glaser, K.B., Adv. Pharmacol. 32: 31-66 (1995);
17. Tykka, H.T. et al., Scand. J. Gastroenterol. 20: 5-12 (1985);
18. Sheuer, W., Klin. Wochenschr. 67: 153-159 (1989);
19. Barnes, H.J. et al., J. Mol. Biol., Feb. 23, 1996;
20. Aoki, A. et al., Mol. Hum. Reprod. 2: 419-497 (1996);
21. Anderson and Kurkland, Microbiological Reviews 54: 198-210 (1990);
22. Miele, L. et al., J. Biol. Chem. 265: 6427-6435 (1990);
23. Coalson, J.J. et al., Exp. Mol. Pathol. 37: 355-360 (1982);
24. Nagy, A. et al., Proc. Natl. Acad. Sci. 90: 8424 (1993);
25. Capecchi, M.R., Science, 244: 1288 (1989);
26. Harlow, E. and Lane D. Antibodies: A Laboratory Manuel, 1st Ed. Cold Spring Harbor Laboratory Press, Cold Spring Harbor, NY, 1988;
27. Mantile, G. et al., J. Biol. Chem. 267: 20343 (1993);
28. Ruoslahti, E. Ann Rev. Biochem. 57: 375 (1988);
29. R.O. Hynes, Fibronectins, New York: Springer-Verlag (1990);
30. Chernousor, M.A. et al., J. Biol. Chem. 266: 10857 (1991);
31. Zhang, Q. et al., J. Cell. Biol. 127: 1447 (1994);
32. Wu, C. et al. Cell 83: 715 (1995);
33. Zhang, Q. et al., J. Biol. Chem. 271: 33284 (1996);
34. Border, W.A. et al., J. Clin. Invest. 90: 1 (1992);
35. Peri, A., et al., J. Clin. Invest. 92: 2099 (1995);
36. Peri, A. et al., J. Clin. Invest. 96: 343 (1995);
37. Oh, E. et al., Proc. Natl. Acad. Sci. (USA) 78: 3218 (1981);
38. Mosher, D.F. et al., Curr, Biol. 4: 810 (1992).

## Claims

1. A pharmaceutical composition comprising a fibronectin binding effective amount of recombinant human uteroglobin (rhUG) or a derivative thereof with the proviso that the derivative retains the biological activity of the parent molecule, a lung surfactant and a pharmaceutically acceptable carrier or diluent.

2. The use of a fibronectin binding effective amount of native or recombinant human uteroglobin (UG) or derivative thereof, with the proviso that the derivative retains the biological activity of the parent molecule, in the manufacture of a pharmaceutical composition for treating or preventing a fibrotic condition in a patient in need of such treatment.

3. The use of a compensating amount of native or recombinant human uteroglobin (UG) or derivative thereof, with the proviso that the derivative retains the biological activity of the parent molecule, in the manufacture of a pharmaceutical composition for treating or preventing a fibrotic condition **characterised by** a deficiency of endogenous uteroglobin.

4. The use of claim 2 or claim 3, wherein said fibrotic condition is pulmonary fibrosis, renal fibrosis or vascular fibrosis.

5. The use of claim 2 or claim 3, wherein said pharmaceutical composition is for the treatment of a fibrotic condition **characterized by** a deficiency of endogenous uteroglobin, wherein said condition is selected from the group consisting of neonatal broncho-pulmonary dyplasia, complications of hemodialysis, bleomycin lung, chronic obstructive pulmonary disease, and inherited glomerulopathy.

6. The use of any one of claims 2 to 5, wherein said UG has a purity of between about 75% to about 100%, preferably between about 90% to about 100%, most preferably at least 95%.

7. The use of any one of claims 2 to 6, wherein said UG is recombinant (rhUG), having substantially the same sequence as native human uteroglobin.

8. The use of any one of claims 2 to 7, wherein said UG is to be administered in association with a lung surfactant.

9. The use of claim 8, wherein said lung surfactant is present in an amount of about 20% to about 80% by weight.

10. The use of any one of claims 2 to 9, wherein said UG is to be administered by the endotracheal, ophthalmic, intravenous, systemic, intraperitoneal, intramuscular, or oral route.

11. The use of any one of claim 2 to 10, wherein said UG is to be administered as a liquid or semi-aerosol via an intratracheal tube.

12. The use of any one of claims 2 to 11, wherein said UG is to be administered in combination with an active agent selected from the group consisting of steroids, nonsteroidal antiinflammatory agents, chemotherapeutics, analgesics, antibodies, antiparasites, antivirals, antibiotics, antifungals, vaccines, tumor vaccines, immunosuppressives, hematopoietic growth factors, antithrombotics, cardiovascular drugs, one or more vitamin and mineral supplements, and fertility drugs.

13. An assay for quantitating uteroglobin-fibronectin complexes in a clinical sample, wherein
(a) a clinical sample suspected of containing one or more uteroglobin-fibronectin complexes is contacted with an antigen capture agent,
(b) an antigen detection agent is added to said sample, and
(c) the presence of any complex bound to said capture agent is detected using a secondary antibody which binds to the antigen detection agent and is conjugated to a moiety that is capable of being detected by the addition of a compound which reacts with said moiety.

14. The assay of claim 13, wherein said antigen capture agent is a monospecific rabbit polyclonal antibody, immobilized on an insoluble support.

15. The assay of claim 13 or claim 14, wherein the secondary antibody is an anti-IgG antibody, the moiety to which it is conjugated is an enzyme, and the compound which reacts with the moiety is a substrate for the enzyme which is converted to a chromogenic or fluorogenic compound upon reaction with the enzyme.

16. The assay of claim 15, wherein the enzyme is horse radish peroxidase.

17. The assay of any one of claims 13 to 16, wherein the antigen detection agent is an antibody specific for fibronectin.

18. The use of a fibronectin binding effective amount of native or recombinant human uteroglobin (UG) or derivative thereof with the proviso that the derivative retains the biological activity of the parent molecule in the manufacture of a pharmaceutical composition for treating or preventing fibrillogenesis in a patient in need of such treatment.

19. The use of claim 18, wherein said UG has a purity of between about 75% to about 100%, preferably between about 90% to about 100%, most preferably at least 95%.

20. The use of claim 18 or claim 19, wherein said UG is recombinant (rhUG), having substantially the same sequence as native human uteroglobin.

21. The use of any one of claims 18 to 20, wherein said UG is to be administered in association with a lung surfactant.

22. The use of claim 21, wherein said lung surfactant is present in an amount of about 20% to about 80% by weight.

23. The use of any one of claims 18 to 22, wherein said UG is to be administered by the endotracheal, ophthalmic, intravenous, systemic, intraperitoneal, intramuscular, or oral routes.

24. The use of any one of claims 18 to 23, wherein said UG is to be administered as a liquid or semi-aerosol via an intratracheal tube.

## Patentansprüche

1. Pharmazeutische Zusammensetzung, umfassend eine wirksame fibronektinbindende Menge an rekombinantem humanem Uteroglobin (rhUG) oder einem Derivat davon mit der Einschränkung, dass das Derivat die biologische Aktivität des Ausgangsmoleküls behält, ein Lungensurfactant und einen pharmazeutisch verträglichen Träger oder Füllstoff.

2. Verwendung einer wirksamen fibronektinbindenden Menge an nativem oder rekombinantem humanem Uteroglobin (UG) oder einem Derivat davon mit der Einschränkung, dass das Derivat die biologische Aktivität des Ausgangsmoleküls behält, zur Herstellung einer pharmazeutischen Zusammensetzung zur Behandlung oder Vorbeugung eines fibrotischen Zustands bei einem behandlungsbedürftigen Patienten.

3. Verwendung einer kompensierenden Menge an nativem oder rekombinantem humanem Uteroglobin (UG) oder einem Derivat davon mit der Einschränkung, dass das Derivat die biologische Aktivität des Ausgangsmoleküls behält, zur Herstellung einer pharmazeutischen Zusammensetzung zur Behandlung oder Vorbeugung eines durch einen Mangel an endogenem Uteroglobin **gekennzeichneten** fibrotischen Zustands.

4. Verwendung nach Anspruch 2 oder Anspruch 3, wobei der fibrotische Zustand Lungenfibrose, Nierenfibrose oder vaskuläre Fibrose ist.

5. Verwendung nach Anspruch 2 oder Anspruch 3, wobei die pharmazeutische Zusammensetzung zur Behandlung eines durch einen Mangel an endogenem Uteroglobin **gekennzeichneten** fibrotischen Zustands ist, wobei der Zustand ausgewählt ist aus der Gruppe bestehend aus neonataler bronchopulmonaler Dysplasie, Hämodialysekomplikationen, Bleomycinlunge, chronisch obstruktiver Lungenerkrankung und angeborener Glomerulopathie.

6. Verwendung nach irgendeinem der Ansprüche 2 bis 5, wobei das UG eine Reinheit zwischen etwa 75 % und etwa 100 %, vorzugsweise zwischen etwa 90 % und etwa 100 %, ganz besonders bevorzugt von wenigstens 95 % besitzt.

7. Verwendung nach irgendeinem der Ansprüche 2 bis 6, wobei das UG rekombinant (rhUG) ist und im Wesentlichen die gleiche Sequenz wie natives humanes Uteroglobin hat.

8. Verwendung nach irgendeinem der Ansprüche 2 bis 7, wobei das UG zur Verabreichung in Verbindung mit einem Lungensurfactant vorgesehen ist.

9. Verwendung nach Anspruch 8, wobei das Lungensurfactant in einer Menge von etwa 20 bis etwa 80 Gew.-% vorliegt.

10. Verwendung nach irgendeinem der Ansprüche 2 bis 9, wobei das UG zur Verabreichung auf endotrachealem, ophthalmologischem, intravenösem, systemischem, intraperitonealem, intramuskulärem oder oralem Wege vorgesehen ist.

11. Verwendung nach irgendeinem der Ansprüche 2 bis 10, wobei das UG zur Verabreichung als Flüssigkeit oder Semi-Aerosol über einen Intratrachealtubus vorgesehen ist.

12. Verwendung nach irgendeinem der Ansprüche 2 bis 11, wobei das UG zur Verabreichung in Kombination mit einem Wirkstoff vorgesehen ist, der ausgewählt ist aus der Gruppe bestehend aus Steroiden, nichtsteroidalen entzündungshemmenden Mitteln, Chemotherapeutika, Analgetika, Antikörpern, Antiparasitika, Antivirusmitteln, Antibiotika, Mitteln gegen Pilze, Impfstoffen, Tumorimpfstoffen, Immunsuppressiva, hämatopoetischen Wachstumsfaktoren, Antithrombotika, kardiovaskulären Arzneimitteln, ein oder mehr Vitamin- und Mineralstoffergänzungsmitteln und Fruchtbarkeitsmitteln.

13. Test zur Quantifizierung von Uteroglobin-Fibronektin-Komplexen in einer klinischen Probe, wobei
(a) eine klinische Probe, von der man vermutet, dass sie ein oder mehrere Uteroglobin-Fibronektin-Komplexe enthält, mit einem Mittel zum Antigen-Capture in Kontakt gebracht wird,
(b) der Probe ein Mittel zum Antigennachweis zugesetzt wird, und
(c) das Vorliegen irgendeines an das Capture-Mittel gebundenen Komplexes mit einem Sekundärantikörper nachgewiesen wird, der an das Mittel zum Antigennachweis bindet und mit einer Komponente konjugiert ist, die durch die Zugabe einer Verbindung nachgewiesen werden kann, die mit der Komponente reagiert.

14. Test nach Anspruch 13, wobei das Mittel zum Antigen-Capture ein monospezifischer polyklonaler Kaninchenantikörper ist, der auf einem unlöslichen Träger immobilisiert ist.

15. Test nach Anspruch 13 oder Anspruch 14, wobei der Sekundärantikörper ein Anti-IgG-Antikörper ist, die Komponente, mit der er konjugiert ist, ein Enzym ist, und die Verbindung, die mit der Komponente reagiert, ein Substrat für das Enzym ist, das bei Reaktion mit dem Enzym in eine chromogene oder fluorogene Verbindung umgewandelt wird.

16. Test nach Anspruch 15, wobei das Enzym Meerrettichperoxidase ist.

17. Test nach irgendeinem der Ansprüche 13 bis 16, wobei das Mittel zum Antigennachweis ein fibronektinspezifischer Antikörper ist.

18. Verwendung einer wirksamen fibronektinbindenden Menge an nativem oder rekombinantem humanem Uteroglobin (UG) oder einem Derivat davon mit der Einschränkung, dass das Derivat die biologische Aktivität des Ausgangsmoleküls behält, zur Herstellung einer pharmazeutischen Zusammensetzung zur Behandlung oder Vorbeugung von Fibrillenbildung bei einem behandlungsbedürftigen Patienten.

19. Verwendung nach Anspruch 18, wobei das UG eine Reinheit zwischen etwa 75 % und etwa 100 %, vorzugsweise zwischen etwa 90 % und etwa 100 %, ganz besonders bevorzugt von wenigstens 95 % besitzt.

20. Verwendung nach Anspruch 18 oder Anspruch 19, wobei das UG rekombinant (rhUG) ist und im Wesentlichen die gleiche Sequenz wie natives humanes Uteroglobin hat.

21. Verwendung nach irgendeinem der Ansprüche 18 bis 20, wobei das UG zur Verabreichung in Verbindung mit einem Lungensurfactant ist.

22. Verwendung nach Anspruch 21, wobei das Lungensurfactant in einer Menge von etwa 20 bis etwa 80 Gew.-% vorliegt.

23. Verwendung nach irgendeinem der Ansprüche 18 bis 22, wobei das UG zur Verabreichung auf endotrachealem, ophthalmologischem, intravenösem, systemischem, intraperitonealem, intramuskulärem oder oralem Wege vorgesehen ist.

24. Verwendung nach irgendeinem der Ansprüche 18 bis 23, wobei das UG zur Verabreichung als Flüssigkeit oder Semi-Aerosol über einen Intratrachealtubus vorgesehen ist.

## Revendications

1. Composition pharmaceutique comprenant une quantité liant la fibronectine efficace d'utéroglobine humaine recombinante (rhUG) ou d'un dérivé de celle-ci, avec la condition que le dérivé conserve l'activité biologique de la molécule mère, et un support ou diluant pharmaceutiquement acceptable.

2. Utilisation d'une quantité liant la fibronectine efficace d'utéroglobine humaine (UG) native ou recombinante ou d'un dérivé de celle-ci, avec la condition que le dérivé conserve l'activité biologique de la molécule mère, dans la fabrication d'une composition pharmaceutique pour traiter ou prévenir une affection de type fibrose chez un patient qui nécessite un tel traitement.

3. Utilisation d'une quantité compensatrice d'utéroglobine humaine (UG) native ou recombinante ou d'un dérivé de celle-ci, avec la condition que le dérivé conserve l'activité biologique de la molécule mère, dans la fabrication d'une composition pharmaceutique pour traiter ou prévenir une affection de type fibrose **caractérisée par** une déficience en utéroglobine endogène.

4. Utilisation selon la revendication 2 ou la revendication 3 où ladite affection de type fibrose est la fibrose pulmonaire, la fibrose rénale ou la fibrose vasculaire.

5. Utilisation selon la revendication 2 ou la revendication 3 où ladite composition pharmaceutique est destinée au traitement d'une affection de type fibrose **caractérisée par** une déficience en utéroglobine endogène, où ladite affection est choisie dans le groupe consistant en la dysplasie broncho-pulmonaire néonatale, les complications de l'hémodialyse, le poumon à la bléomycine, la bronchopneumopathie chronique obstructive et la glomérulopathie héréditaire.

6. Utilisation selon l'une quelconque des revendications 2 à 5 où ladite UG a une pureté entre environ 75 % et environ 100 %, de préférence entre environ 90 % et environ 100 %, de manière particulièrement préférable d'au moins 95 %.

7. Utilisation selon l'une quelconque des revendications 2 à 6 où ladite UG est de la (rhUG) recombinante ayant sensiblement la même séquence que l'utéroglobine humaine native.

8. Utilisation selon l'une quelconque des revendications 2 à 7 où ladite UG est destinée à être administrée en association avec un surfactant pulmonaire.

9. Utilisation selon la revendication 8 où ledit surfactant pulmonaire est présent en une quantité d'environ 20 % à environ 80 % en masse.

10. Utilisation selon l'une quelconque des revendications 2 à 9 où ladite UG est destinée à être administrée par la voie endotrachéale, ophtalmique, intraveineuse, systémique, intrapéritonéale, intramusculaire ou orale.

11. Utilisation selon l'une quelconque des revendications 2 à 10 où ladite UG est destinée à être administrée sous forme d'un liquide ou d'un semi-aérosol par le biais d'un tube intratrachéal.

12. Utilisation selon l'une quelconque des revendications 2 à 11 où ladite UG est destinée à être administrée en combinaison avec un agent actif choisi dans le groupe consistant en les stéroïdes, les agents anti-inflammatoires non stéroïdiens, les agents chimiothérapeutiques, les analgésiques, les anticorps, les antiparasitaires, les antiviraux, les antibiotiques, les antifongiques, les vaccins, les vaccins antitumoraux, les immunosuppresseurs, les facteurs de croissance hématopoïétiques, les antithrombotiques, les médicaments cardiovasculaires, un ou plusieurs compléments vitaminiques et minéraux, et les médicaments de fécondité.

13. Dosage pour quantifier les complexes utéroglobine-fibronectine dans un échantillon clinique, où
(a) un échantillon clinique suspecté de contenir un ou plusieurs complexes utéroglobine-fibronectine est mis en contact avec un agent de capture d'antigène,
(b) un agent de détection d'antigène est ajouté audit échantillon, et
(c) la présence de tout complexe lié audit agent de capture est détectée au moyen d'un anticorps secondaire qui se lie à l'agent de détection d'antigène et est conjugué à une entité qui peut être détectée par addition d'un composé qui réagit avec ladite entité.

14. Dosage selon la revendication 13 où ledit agent de capture d'antigène est un anticorps polyclonal de lapin monospécifique, immobilisé sur un support insoluble.

15. Dosage selon la revendication 13 ou la revendication 14 où l'anticorps secondaire est un anticorps anti-IgG, l'entité à laquelle il est conjugué est une enzyme, et le composé qui réagit avec l'entité est un substrat pour l'enzyme qui est converti en un composé chromogène ou fluorogène par réaction avec l'enzyme.

16. Dosage selon la revendication 15 où l'enzyme est la peroxydase de raifort.

17. Dosage selon l'une quelconque des revendications 13 à 16 où l'agent de détection d'antigène est un anticorps spécifique à l'égard de la fibronectine.

18. Utilisation d'une quantité liant la fibronectine efficace d'utéroglobine humaine (UG) native ou recombinante ou d'un dérivé de celle-ci, avec la condition que le dérivé conserve l'activité biologique de la molécule mère, dans la fabrication d'une composition pharmaceutique pour traiter ou prévenir la fibrillogenèse chez un patient qui nécessite un tel traitement.

19. Utilisation selon la revendication 18 où ladite UG a une pureté entre environ 75 % et environ 100 %, de préférence entre environ 90 % et environ 100 %, de manière particulièrement préférable d'au moins 95 %.

20. Utilisation selon la revendication 18 ou la revendication 19 où ladite UG est la (rhUG) recombinante ayant sensiblement la même séquence que l'utéroglobine humaine native.

21. Utilisation selon l'une quelconque des revendications 18 à 20 où ladite UG est destinée à être administrée en association avec un surfactant pulmonaire.

22. Utilisation selon la revendication 21 où ledit surfactant pulmonaire est présent en une quantité d'environ 20 % à environ 80 % en masse.

23. Utilisation selon l'une quelconque des revendications 18 à 22 où ladite UG est destinée à être administrée par la voie endotrachéale, ophtalmique, intraveineuse, systémique, intrapéritonéale, intramusculaire ou orale.

24. Utilisation selon l'une quelconque des revendications 18 à 23 où ladite UG est destinée à être administrée sous forme d'un liquide ou d'un semi-aérosol par le biais d'un tube intratrachéal.


### SEQUENCE LISTING

<110> Pilon, Aprile, et al.
<120> USE OF RECOMBINANT HUMAN UTEROGLOBIN IN TREATMENT OF INFLAMMATORY AND FIBROTIC CONDITIONS
<130> SEQUENCE LISTING
<140> 08/864,357
   <141> 1997-05-28
<160> 13
<170> PatentIn Ver. 2.0
<210> 1
   <211> 70
   <212> PRT
   <213> Artificial Sequence
<220>
   <223> Description of Artificial Sequence: human uteroglobin
<400> 1
   Glu Ile Cys Pro Ser Phe Gln Arg Val Ile Glu Thr Leu Leu Met Asp
    1               5                  10                  15
   Thr Pro Ser Ser Tyr Glu Ala Ala Met Glu Leu Phe Ser Pro Asp Gln
               20                  25                  30
   Asp Met Arg Glu Ala Gly Ala Gln Leu Lys Lys Leu Val Asp Thr Leu
           35                  40                  45
   Pro Gln Lys Pro Arg Glu Ser Ile Ile Lys Leu Met Glu Lys Ile Ala
       50                  55                  60
   Gln Ser Ser Leu Cys Asn
   65                  70
<210> 2
   <211> 69
   <212> PRT
   <213> Artificial Sequence
<220>
   <223> Description of Artificial Sequence: rabbit uteroglobin
<400> 2
   Gly Ile Cys Pro Arg Phe Ala His Val Ile Glu Asn Leu Leu Leu Gly
   1 5 10 15
   Thr Pro Ser Ser Tyr Glu Thr Ser Leu Lys Glu Phe Glu Pro Asp Asp
   20 25 30
   Thr Met Lys Asp Ala Gly Met Gln Met Lys Lys Val Leu Asp Ser Leu
   35 40 45
   Pro Gln Thr Thr Arg Glu Asn Ile Met Lys Leu Thr Glu Lys Ile Val
   50 55 60
   Lys Ser Pro Leu Cys
   65
<210> 3
   <211> 75
   <212> PRT
   <213> Artificial Sequence
<220>
   <223> Description of Artificial Sequence: rat uteroglobin
<400> 3
   Asp Ile Cys Pro Gly Phe Leu Gln Val Leu Glu Ala Leu Leu Leu Gly
   1 5 10 15
   Ser Glu Ser Asn Tyr Glu Ala Ala Leu Lys Pro Phe Asn Pro Ala Ser
   20 25 30
   Asp Leu Gln Asn Ala Gly Thr Gln Leu Lys Arg Leu Val Asp Thr Leu
   35 40 45
   Pro Gln Glu Thr Arg Ile Asn Ile Val Lys Leu Thr Glu Lys Ile Leu
   50 55 60
   Thr Ser Pro Leu Cys Glu Gln Asp Leu Arg Val
   65 70 75
<210> 4
   <211> 75
<212> PRT
   <213> Artificial Sequence
<220>
   <223> Description of Artificial Sequence: mouse uteroglobin
<400> 4
   Asp Ile Cys Pro Gly Phe Leu Gln Val Leu Glu Ala Leu Leu Met Glu
   1 5 10 15
   Ser Glu Ser Gly Tyr Val Ala Ser Leu Lys Pro Phe Asn Pro Gly Ser
   20 25 30
   Asp Leu Gln Asn Ala Gly Thr Gln Leu Lys Arg Leu Val Asp Thr Leu
   35 40 45
   Pro Gln Glu Thr Arg Ile Asn Ile Met Lys Leu Thr Glu Lys Ile Leu
   50 55 60
   Thr Ser Pro Leu Cys Lys Gln Asp Leu Arg Phe
   65 70 75
<210> 5
   <211> 23
   <212> DNA
   <213> Artificial Sequence
<220>
   <223> Description of Artificial Sequence: primer-L
<400> 5
   ttccaaggca gaacatttga gac 23
<210> 6
   <211> 21
   <212> DNA
   <213> Artificial Sequence
<220>
   <223> Description of Artificial Sequence: primer-R
<400> 6
   tctgagccag ggttgaaagg c 21
<210> 7
   <211> 23
   <212> DNA
   <213> Artificial Sequence
<220>
   <223> Description of Artificial Sequence: primer
<400> 7
   atcttgctta cacagaggac ttg 23
<210> 8
   <211> 20
   <212> DNA
   <213> Artificial Sequence
<220>
   <223> Description of Artificial Sequence: primer
<400> 8
   atcgccatca caatcactgt 20
<210> 9
   <211> 25
   <212> DNA
   <213> Artificial Sequence
<220>
   <223> Description of Artificial Sequence: primer
<400> 9
   atcagagtct ggttatgtgg catcc 25
<210> 10
   <211> 20
   <212> DNA
   <213> Artificial Sequence
<220>
   <223> Description of Artificial Sequence: primer
<400> 10
   ggcatcgaag gtggaagagt 20
<210> 11
   <211> 20
   <212> DNA
   <213> Artificial Sequence
<220>
   <223> Description of Artificial Sequence: primer
<400> 11
   atggccttcc gtgttcctac 20
<210> 12
   <211> 26
   <212> DNA
   <213> Artificial Sequence
<220>
   <223> Description of Artificial Sequence: primer
<400> 12
   gaaggtggtg aagcaggcat ctgagg 26
<210> 13
   <211> 22
   <212> PRT
   <213> Artificial Sequence
<220>
   <223> Description of Artificial Sequence: mouse uteroglobin amino acid sequence
<400> 13
   Lys Pro Phe Asn Pro Gly Ser Asp Leu Gln Asn Ala Gly Thr Gln Leu
    1               5                  10                  15
   Lys Arg Leu Val Asp Thr
               20